# EUROPEAN PATENT SPECIFICATION

(11) **EP 1 261 253 B1**
(45) Date of publication and mention of the grant of the patent: **23.09.2009**
(21) Application number: 01914006.0
(22) Date of filing: 12.03.2001
(51) Int. Cl.: A01K 5/02, A23K 1/00, C12N 11/00, C12N 1/04

(54) **STORAGE AND DELIVERY OF MICRO-ORGANISMS**
LAGERUNG UND ABGABE VON MIKROORGANISMEN
STOCKAGE ET ADMINISTRATION DE MICRO-ORGANISMES

(30) Priority: 10.03.2000 GB 0005839; 20.03.2000 US 190858 P
(43) Date of publication of application: 04.12.2002
(60) Divisional application: 03078156.1
(73) Proprietor: Uutech Limited, Coleraine, County Londonderry, BT52 1SA (GB)
(72) Inventor: MCGRATH, Susan, New Haven, CT 06511 (US); MCHALE, Anthony Patrick, Coleraine, County Londonderry BT52 1SA (GB)
(74) Representative: O'Connell, Maura
(86) International application number: PCT/GB2001/001062
(87) International publication number: WO 2001/065923

(56) References cited:
- EP-A- 0 203 708
- EP-A- 0 259 739
- EP-A- 0 323 782
- EP-A- 1 005 788
- FR-A- 2 714 575
- FR-A- 2 748 752
- US-A- 4 138 498
- US-A- 5 283 059
- US-A- 6 033 887

## Description

### FIELD OF THE INVENTION

This invention relates to a method and an apparatus for delivering of micro-organisms to animals, particularly probiotics in animal feed and/or a drinking water system.

### BACKGROUND TO THE INVENTION

It has been known for many years that certain strains of micro-organisms impart beneficial effects via the digestive tract of both animals and humans. Such micro-organisms are known as probiotics. The benefits of employing microbial probiotics and the scientific basis thereof have been reviewed by Ried (1999. Appl. Env. Microbiol 65, 3763-3766).

Much of the research done on the use and benefits of microbial probiotics has been conducted in *Lactobacillus* strains. Beneficial effects arise from an increased concentration of the *Lactobacillus* strains in the digestive tract. The benefits include reduced diarrhoea in calves (Chaves et al., 1999, Braz. J. Animal Sci. 28, 1093-1101). reduced colonisation by *Salmonella* in chicks (Singh et al., 1999, Appl. Env. Microbiol. 65, 4981-4986), reduced overall mortality in chicks (Singh et al, 1999. Ind. J. Animal Sci., 69, 830-831) and reduced assimilation of cholesterol in cocks fed on a high cholesterol diet (Endo et al., 1999. Biosci. Biotech. Biochem. 63. 1569-1575). It appears that many of the beneficial effects observed with *Lactobacillus* probiotic strains result from colonisation of the digestive tract by those strains, thereby precluding colonisation by other more detrimental micro-organisms. It has been found that, for these beneficial effects to persist, the probiotic strains must be supplied to the host on a continuous basis to ensure persistence in the digestive tract.

In addition to *Lactobacillus* strains, other micro-organisms, most notably including the *Enterococci,* have been shown to exhibit probiotic activity. In the case of *Enterococci,* the beneficial effects appear to be linked not only to preferential colonisation but also to their ability, in certain cases, to produce bacteriocins. It has been reported, for example, that some strains produce bacteriocins that exhibit anti-listerial effects (Laukova et al., 1998, Letts. Appl. Microbiol. 27, 178-182).

The beneficial effects of these micro-organisms may be controlled or enhanced by increasing their presence or concentration in the digestive tract (Reid, 1999, Appl. Env. Microbiol., 65; 3763-3766). This may be accomplished by modifying the preparation of the animal feed-stuff to maximise the concentration of endogenous beneficial micro-organisms, for example, by ensilation. In ensilation, conditions are manipulated to ensure a rapid decrease in pH, thereby enhancing the persistence of the beneficial lactic acid bacterial strains (LABs) and preventing growth of detrimental anaerobic micro-organisms such as *Clostridia* (Lindgren et al., 1986; Swed. J. Agric. Res., 15: 9-18).

Alternatively, probiotic micro-organisms may be delivered to the animal by adding the relevant microbial strain to animal feed-stuff. Typically, the micro-organism is added in dry form to feed-stuff, and is taken up together with the feed stuff during feeding. Alternatively, micro-organisms may be added to the animals' drinking water. An amount of the probiotic micro-organism, which may be in the form of a cake, is weighed and mixed with the feed-stuff or drinking water. It is, however, often difficult to ensure that the correct amount of probiotic has been added each time or that the probiotic has been added at all. Furthermore, unless the probiotic is thoroughly mixed with feed-stuff or drinking water, the probiotic will not be evenly distributed throughout the feed-stuff or drinking water, and problems will occur.

There is therefore a need for a system of delivering probiotic micro-organisms to animals which overcomes the problems associated with traditional methods.

### SUMMARY OF THE INVENTION

We have now found that it is possible to immobilise micro-organisms in a matrix so that they remain viable during storage for extended periods of time. Subsequently, the micro-organisms may be detached from the matrix and mixed into a feed stream for the animal. The micro-organisms may then be ingested by the animal when it feeds on the feed stream.

In accordance with a first aspect of the invention, we provide a method of delivering a micro-organism to an animal according to claim 1.

Preferably, the micro-organisms which are detached are conveyed to the feed stream to be entrained therewith. Alternatively, the micro-organisms may be detached and entrained by contacting the formulation with the feed stream.

In accordance with a second aspect of the invention, we provide an apparatus for delivering a micro-organism to an animal according to claim 2.

The apparatus may further comprise means for conveying detached micro-organisms to the feed stream. Alternatively, the apparatus may allow direct contact between the formulation and the feed stream, so that relative motion between the feed stream and the formulation causes micro-organisms to be detached and entrained into the feed stream. The apparatus may further comprise means for delivering micro-organisms to the feed stream in a batch, pulsed or continuous flow manner, or in any combination of these. Preferably, the formulation is such that micro-organisms are capable of being detached from the formulation in a substantially continuous manner.

The formulation comprises a source of probiotic micro-organisms suspended in a matrix, the formulation preferably being capable of allowing micro-organisms to be detached therefrom in a substantially continuous or semi-continuous manner.

The suspended micro-organisms act as an innoculative source of the micro-organism for the animal feed or drinking water. Where reference is made to micro-organisms being detached from the matrix, we mean the release, separation or detachment of micro-organisms from the matrix. The detached micro-organisms may include descendants of the originally suspended micro-organisms. Thus, the suspended micro-organisms (and/or descendants thereof) may be physically freed from the matrix. Alternatively, daughter cells of originally suspended micro-organisms are detached from the surface of the matrix.

The micro-organisms may be detached by mechanical action on the formulation, for example, by being sloughed off. Alternatively or in conjunction, the micro-organisms may be detached by the molecules forming the matrix being dissolved in the environment. The formulation may also be added directly to the feed stream and be taken in by the animal with the feed or water.

Preferably, the micro-organism is a probiotic organism. The term "probiotic" is known in the art, and refers to a live microbial feed supplement which beneficially affects the host animal by improving its intestinal microbial balance. Examples of probiotic micro-organisms are *Bifidobacterium, Lactococcus, Lactobacillus* and *Enterococcus.*

Preferred probiotic micro-organisms are *Lactobacillus* or *Enterococcus spp.* More preferably, the probiotic micro-organism is selected from *Lactobacillus acidophilus* LA-107 (ATCC no. 53545), *Laccobacillus acidophilus* LA-101 (ATCC no. 4356) and *Enterococcus faecium* EF-101 (ATCC no. 19434).

The formulation may comprise micro-organisms all of which comprise the same strain or species. Alternatively and preferably, the formulation comprises micro-organisms of two or more strains or species. Recombinant forms of any of the micro-organisms may also be used, whether alone or in combination with non-recombinant or wild type forms.

The formulation may comprise beads which immobilise the micro-organism. The formulation may comprise a cartridge containing a plurality of beads. The beads may be micro-beads or micro-spheres. Preferably, the beads have a diameter between about 2 and 3 millimetres.

The matrix preferably comprises a water porous material, preferably a gel. Preferably, the micro-organisms are immobilised in the matrix. The matrix may comprise agar, agarose, starch, carrageenan, locust bean gum, poly(vinyl) alcohol based gels or cryogels (Gough et al. (1998) Bioprocess Eng. 19, 87-90), siliceous fossil meals (for example, kieselguhr, tripolite and diatomite) or volcanic ash such as kissiris (Love et al. (1998) Bioprocess Eng.18, 187-189). The matrix may comprise any suitable support, and may be made of, for example, plastics, including biodegradeable plastics. The matrix may further comprise polylactides (Huang et al., 1999, Int. J. Pharm., 182, 93-100), polymers comprising lactic acid subunits, polyglycolides, polylactide-polyglycolide, poly(D,L lactide co glycolide) (Seifert, et al., 1997, Biomaterials, 18, 1495-502), or block co-polymers or hydrogel block co-polymers for example comprising repeating subunits of 2 hydroxyethylmethacrylate and ethylene dimethacrylate (Wheeler et al., 1996, J. Long Term Eff Med Implants, 6, 207-217). Indeed any material derived from a plant or animal material or artificial, synthetic materials such as plastic, paper, etc may be used.

Preferably, the matrix comprises an alginate. The term "alginate" refers to a copolymer of beta-D-mannuronic acid and alpha-L-guluronic acid (GulA), linked together by 1-4 linkages. Alginates, together with their esters and metallic salts. comprise the principal carbohydrate component of the brown seaweeds *Ascophyllum, Laminaria* and *Macrosystis.* Preferably, the alginate is an alkaline earth metal alginate, and most preferably the alginate is a calcium or barium alginate.

The formulation may also comprise nutrients, additives or supplements to aid the growth of the animals being fed, such as natural or synthetic growth enhancers, growth hormones, recombinant products, vitamins, micronutrients, antibiotics, etc. Other examples of such nutrients, etc are known in the art. The formulation may, instead of or in addition to the nutrients, additives or supplements, include micro-organisms capable of producing these nutrients etc.

By "feed stream", we mean a substantially continuous train, trail or stream of food, feedingstuffs, feed material, water, or other solid, semi-solid or liquid nutrient which is conveyed from a source. The feedingstream may comprise any products of vegetable or animal origin, organic or inorganic substances and may be in their natural state, fresh or preserved, industrially processed, mixtures of any of these etc. The feed stream is preferably conveyed to a feeding point for the animal. The feed stream is preferably a moving feed stream, which may be delivered in a continuous or semi-continuous fashion. The feed stream may be provided on a conveyor belt or drain, and this is suitable where the food or nutrient is in solid form. The food or other nutrient may be carried in containers, for example, buckets disposed on a conveyor belt. Where feed stream carries water, liquid food or semi-liquid food, the feed stream is preferably provided in a pipe, gully or other conduit. Preferably, the feed stream comprises drinking water for the animal.

Preferably, the formulation is provided in a form which is substantially free of water. The formulation may be freeze-dried, lyophilised or spray dried, or otherwise dried by methods known in the art. Accordingly, the formulation preferably comprises a preservative capable of maintaining the viability of the probiotic micro-organisms, so a substantial proportion of the micro-organisms are alive when the formulation is kept for extended periods of time. Preferably, the preservative is a cryopreservant. Preferably, the preservative comprises trehalose or lactose.

Although trehalose has been reported to be very efficient in protecting biological material from dehydration, we have found that lactose is just as efficient as trehalose in preserving micro-organisms. Accordingly, and most preferably, the preservative or cryopreservant is lactose. The micro-organism may be genetically engineered to express a trehalose biosynthesis enzyme or a lactose biosynthesis enzyme, for example by introduction of *Escherichia coli* otsA and otsB genes. Thus, the formulation may be kept or stored for extended periods of time in a dry, semi-dry or moist state without compromising the viability of the micro-organisms. This is advantageous in that it allows the formulation to be easily stored.

### BRIEF DESCRIPTION OF THE DRAWINGS

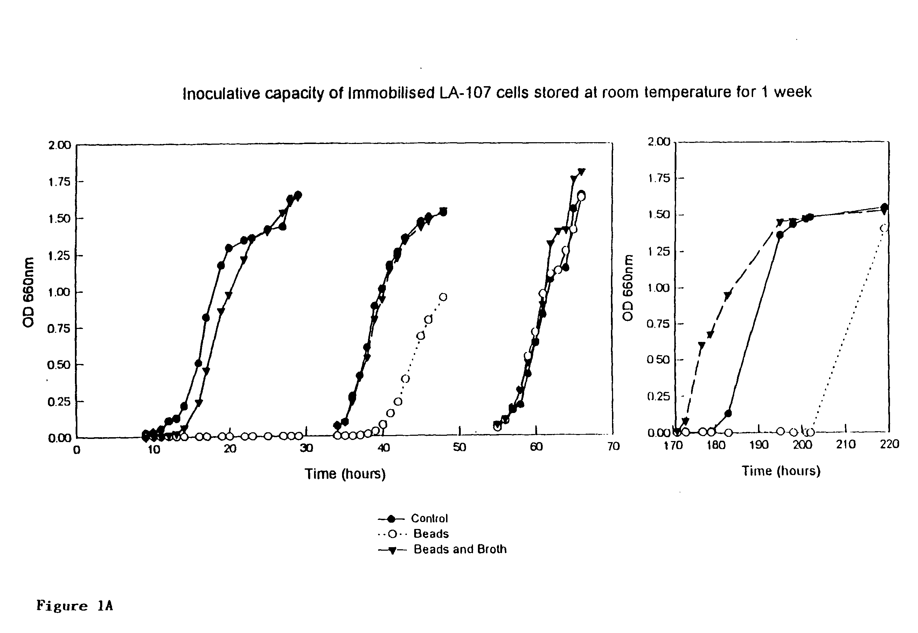

Figure 1A shows the results of an experiment to determine the inoculative capacity of immobilised *Lactobacillus acidophilus* LA-107 cells which have been stored at room temperature for a week. Broth samples are taken and the absorbance at 660nm measured and plotted against time.

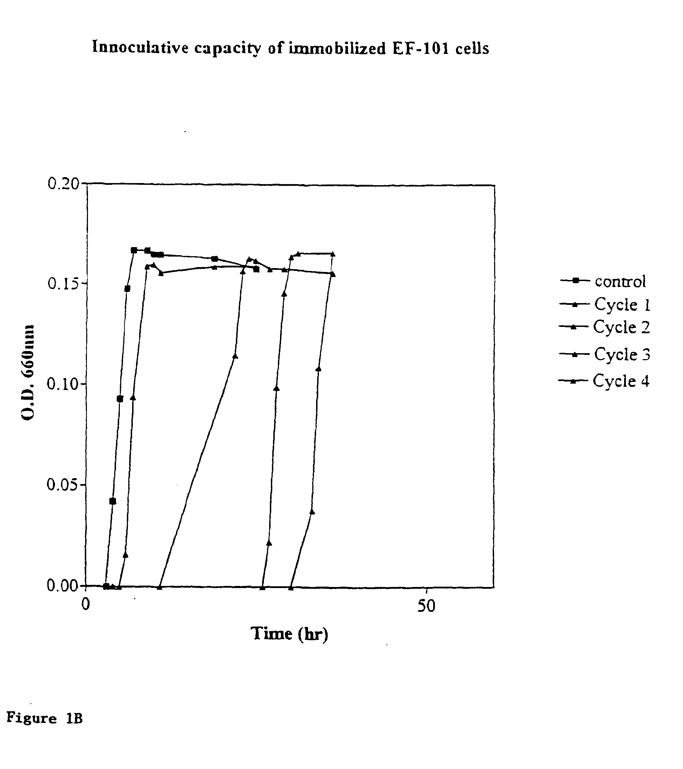

Figure 1B shows the results of a similar experiment conducted with immobilised *Enterococcus faecium* EF-101 cells. Cycle I indicates that refeeding is carried out at 7 hours, cycle 2 indicates that refeeding is carried out at 19 hours, cycle 3 indicates that refeeding is carried out at 26 hours, while cycle 4 indicates that refeeding is carried out at 32 hours.

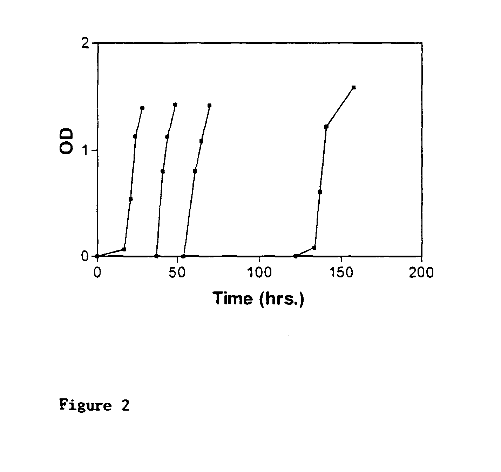

Figure 2 shows the inoculating capacity of immobilised formulations prepared by firstly growing the microorganism in medium together with sodium alginate and subsequent addition of the fermented mixture directly to calcium chloride. Fresh medium is added to beads at T = 0. Spent medium is then replaced by fresh medium at 37, 54 and 122 hours. The x-axis denotes time measured in hours and the y-axis denotes the optical density (absorbance) at 660nm.

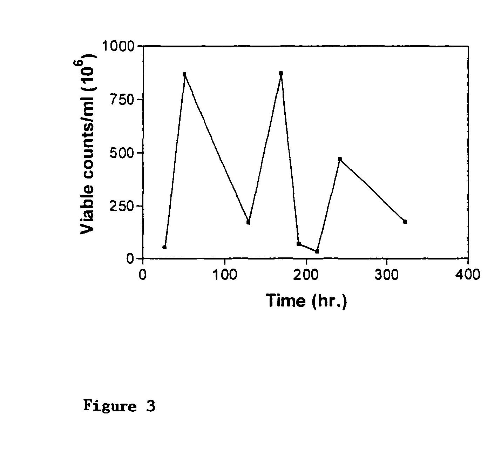

Figure 3 shows the continuous production of probiotic from a fixed-bed reactor system containing kelp as the immobilisation medium. The x-axis denotes time measured in hours and the y-axis denotes the viable counts x 10⁶/ml in effluents form the reactor.

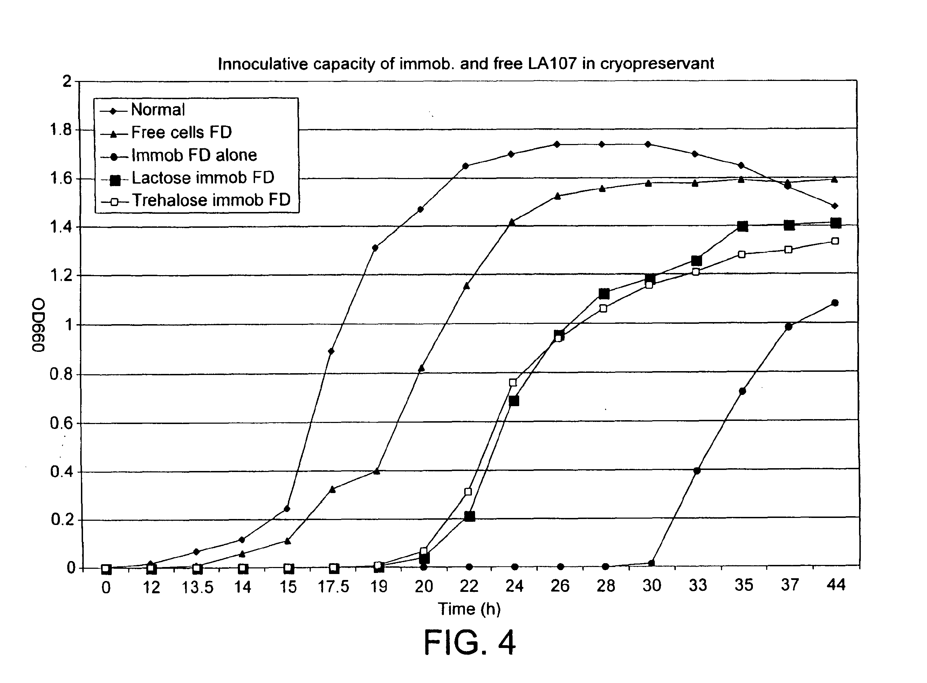

Figure 4 shows results of an experiment to show the innoculative capacity of *Lactobacillus acidophilus* LA-107 cells which have been immobilised in the presence of various preservatives and/or cryopreservants. Absorbance at 660 nm is plotted against time. "FD" indicates that the cells have been freeze dried.

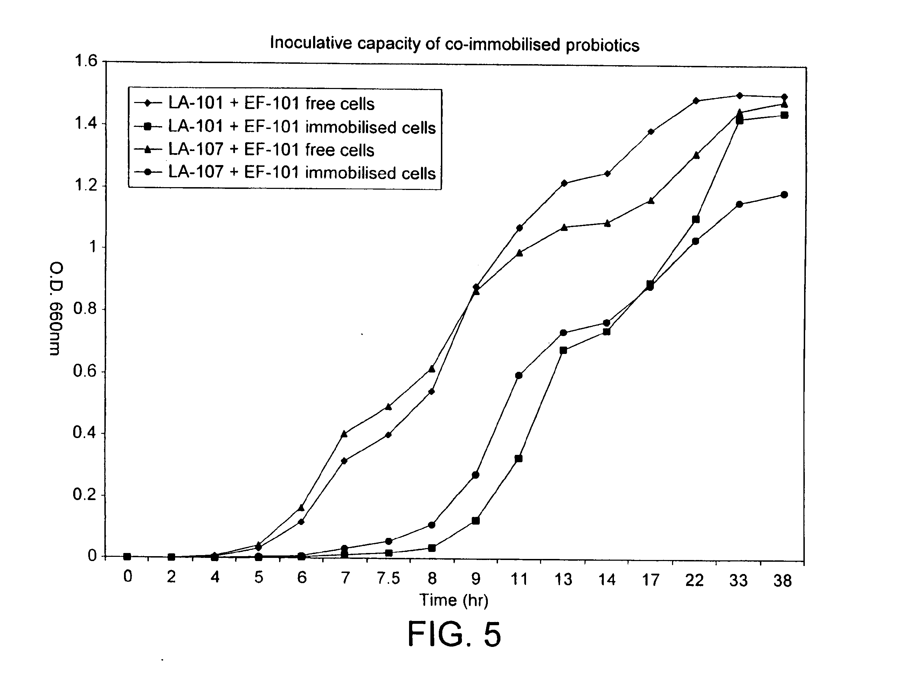

Figure 5 shows the inoculative capacity of co-immobilised products. The graph shows results of experiments in which two different microbial strains are immobilised in an alginate matrix. The graph plots cell density (measured by absorbance of the culture medium) against time.

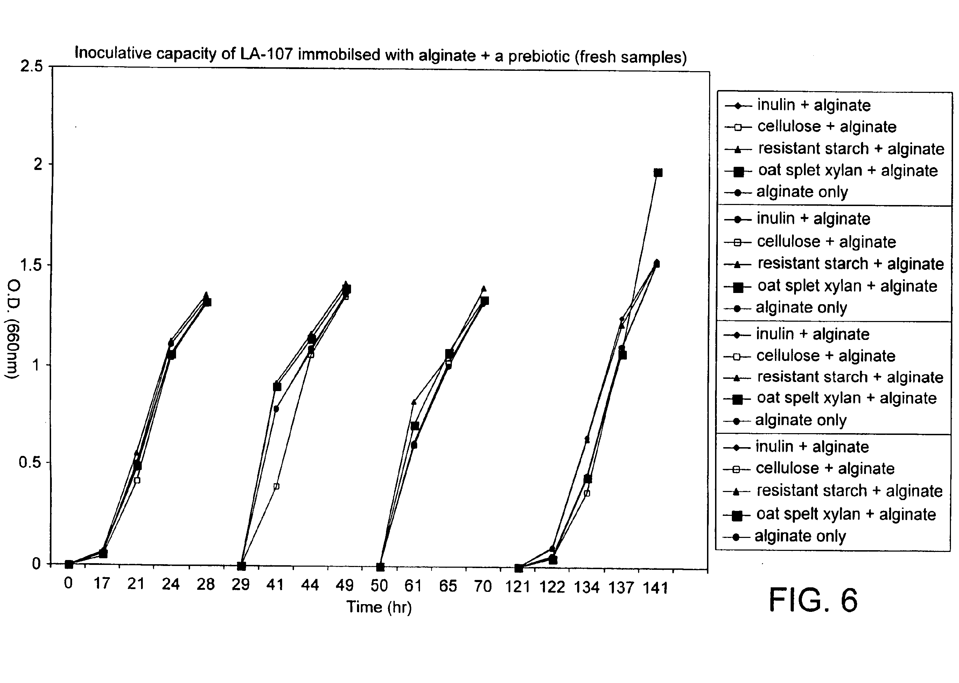

Figure 6 shows the inoculating capacity of beads in which the probiotic microorganism *Lactobacillus acidophilus* strain 107 is co-immobilised together with inulin, cellulose, resistant starch and oat spelt xylan (pre-biotics). The control consists of microorganism immobilised in alginate alone (filled circles). Beads are fed with fresh medium at T = 0 and spent medium is removed and replaced with fresh medium at 29. 50 and 121 hours. The x-axis denotes time measured in hours and the y-axis denotes the optical density (absorbance) at 660nm.

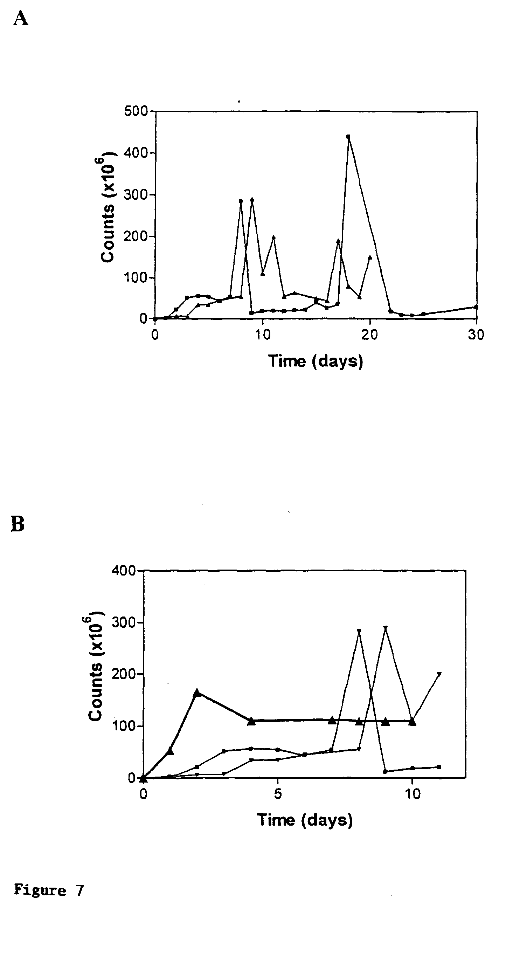

Figures 7A shows continuous production of probiotic microorganism from a fixed-bed reactor. Counts x 10⁶ produced by two separate runs (filled triangles and filled squares) are determined by direct counting. Figure 7B shows a comparison of viable microorganism from the reactor operated in a fixed-bed (inverted filled triangles and filled squares) and fluidised bed (filled triangles) configuration. In both cases the x-axis denotes time measured in days and the y-axis denotes viable counts x 10⁶/ml in effluents from the relevant reactor.

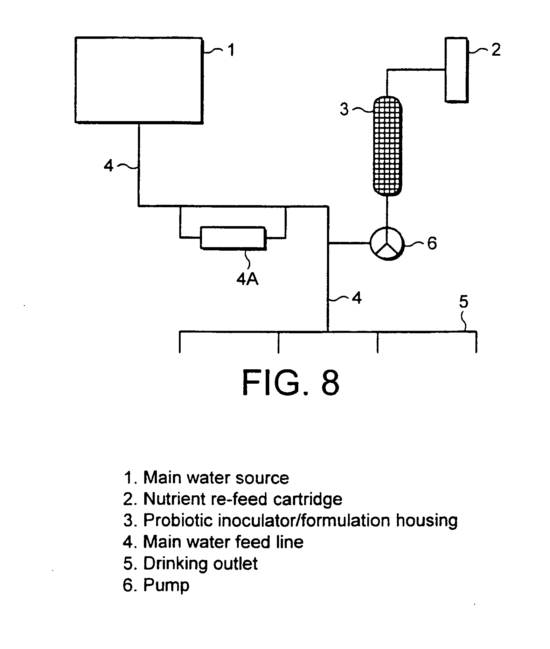

Figure 8 is a schematic diagram of an apparatus for delivering a probiotic micro-organism to an animal.

### DETAILED DESCRIPTION OF THE INVENTION

Our invention allows a source of probiotic micro-organisms to be continuously provided into the food chain of an animal. The probiotic micro-organisms are provided as a formulation comprising cells suspended or immobilised in a matrix.

The probiotic micro-organism may comprise any species or strain which is known to have beneficial effects to the animal. The probiotic micro-organism may be an *Enterococcus,* for example, *Enterococcus faecium, Enterococcus faecium* PR88, *Enterococcus casseliflavus, Enterococcus faecalis, Enterococcus faecalis* V24, *Enterococcus avium* or *Enterococcus durans.* The probiotic micro-organism may also be a *Lactobacillus,* for example, *Lactobacillus animalis, Lactobacillus fermentum, Lactobacillus animalis subsp cellobiosus, Lactobacillus acidophilus* strains LT516 (Chaves et al, 1999, Brazilian Journal ofAnimal Science, 28, 1075-1085) and LT158A (Chaves et al., 1999, Brazilian Journal of Animal Science, 28, 1093-1101), *Lactobacillus salivarius* CTC2197 (Pascual et al, 1999, Applied and Environmental Microbiology, 65, 4981-4986), *Lactobacillus sporogenes* (Singh et al, 1999, Indian Journal of Animal Sciences, 69, 830-831), *Lactobacillus bifidus, Lactobacillus leichmannii, Lactobacillus plantarum, Lactobacillus casei, Lactobacillus helveticus* CNRZ-303, *Lactobacillus delbruckii* subsp *bulgaricus-*12, *Lactobacillus helveticus* INF-II, *Lactobacillus plantarum* INF-9a, *Lactobacillus casei* subsp *casei* INF-15d, *Lactobacillus casei* subsp *pseudocasei* INF-13i, or *Lactobacillus fermentum.* The probiotic microorganism may also be a *Bifidobacterium,* for example, *Bifidobacterium bifidum.*

It will be appreciated that recombinant forms of any of the above micro-organisms may also be used. Indeed, where reference is made to a "micro-organism" in this document, such reference is to be understood as encompassing recombinant variants of the micro-organism. Such recombinant forms may comprise engineered forms for altered characteristics, expression of useful products, better survival, etc.

Preferred probiotic micro-organisms are those which have been approved by the relevant regulatory authorities for use. Such micro-organisms include those annexed in, for example, European Council Directive 70/524/EEC of 23 November 1970 concerning additives in feeding-stuffs (Official Journal L 270 , 14/12/1970 p. 0001 - 0017), or as amended by subsequent legislation (see http://europa.eu.int/eurlex/en/lif/dat/1970/en_370L0524.html). A list of such micro-organisms is provided in Annex I of this document.

The matrix may be any matrix capable of preserving substantial viability of the micro-organisms, so that a substantial number of micro-organisms are alive when the formulation is kept for extended periods of time. Preferably, the matrix is chosen so that the viability of suspended micro-organisms is comparable to, or better than, free cells (in other words, cells which have not been immobilised).

A number of matrices have been disclosed as being capable of preserving cells. For example, cells have been suspended in agar, agarose or an agar-agarose mixture, by melting the agar or agarose or mixture. Cells are added to the melted liquid and mixed to form a uniform suspension. The melted agar/agarose/mixture is then cooled to below the relevant melting point.

Alternatively, and as a preferred embodiment, the probiotic cells are grown in an appropriate culture medium as known in the art. Cells are preferably harvested, for example, by centrifugation and washed. The cells are then suspended in a solution of a soluble alginate salt. It is known that alkali metal alginates are soluble: preferred alginate salts therefore include alkali metal alginates, such as lithium, sodium, potassium, rubidium, etc. Ammonium alginate may also be used. Preferably, sodium alginate is used, at a preferred concentration of 4% weight/volume. The suspension is then added to a solution containing calcium ions to cause a gel to form. Alternatively, the cells may be suspended in the calcium-containing solution and added to a soluble alginate solution. The gel comprises calcium alginate, and contains immobilised probiotic cells suspended in the alginate. If the micro-organism suspension is added dropwise, then the gel forms as beads which may be harvested. The gelled alginate may if necessary be moulded into a block, or formed in layers on a solid support.

A method of immobilisation of cells in calcium alginate capsules is disclosed in US Patent No 5,766,907. Other insoluble alginates which may be used to encapsulate or immobilise the probiotic cells include alginate salts of alkaline earth metals, such as magnesium, calcium, strontium, barium etc alginates. Alginates of other divalent cations may also be employed. Matrixes or gels comprising such alginates may be made by the methods described here.

The preferred embodiment described above employs harvested cells suspended in a solution of a soluble alginate salt. This separation step, although preferred, is not strictly necessary. We have found that it is possible to supplement the growing medium with soluble alginate, and that the micro-organisms remain capable of growing in the alginate containing medium. Beads comprising immobilised or encapsulated micro-organisms may be formed by adding the medium (containing cells and alginate) into a calcium or other solution as described above. Furthermore, micro-organisms may be grown in medium comprising gelling agent such as calcium ions (or other divalent or alkaline earth metal ions, etc), and added into a soluble alginate containing solution to form gels or beads comprising encapsulated micro-organisms.

The cells of the probiotic micro-organism may also be encapsulated in microspheres or microcapsules, by methods known in the art, for example as disclosed in US Patent No. 4.803.168. Microcapsules containing the micro-organisms may also be formed by internal gelation of an alginate solution emulsified with an oil, for example, as described in Esquisabel et al. (1997), Journal of Microencapsulation, 14, 627-638. Alternatively, carrageenan-chitosan gels may be used to immobilise the micro-organisms (Wang and Qian, 1999, Chemosphere, 38, 3109-3117). Biocompatible capsules consisting of a liquid starch core with calcium alginate membranes have also been used to encapsulate lactic acid bacteria (Jankowski et al, 1997. Biotechnology Techniques, 11, 31-34). Barium alginate capsules may also be used instead of calcium (Yoo et al, 1996, Enzyme and Microbial Technology, 19, 428-433).

Other alternative forms of the matrix encapsulating micro-organisms (besides beads) are also possible. For example, cryogels comprising poly(vinyl alcohol) may be employed. Such cryogels are made by providing a solution of a polyvinyl alcohol containing the micro-organism of interest. The solution is then added (for example, dropwise) to a non-miscible solvent such as hexane (or the hexane added to the micro-organism containing solution). Frozen beads form and these, when thawed very slowly (for example, overnight in a fridge) form insoluble beads with viable micro-organism. Such a method may be employed with any soluble polymer, e.g. soluble alginate, carboxymethyl cellulose, polyacrylamide, etc. Reference is made to Gough et al., 1998, Bioprocess Eng., 87-90.

Various matrix shapes may also be used, allowing for alternative reactor configurations. Thus, in addition to beads, the matrix may take the form of discs, blocks, extruded fibres or fibrils, tubes. Furthermore, the matrix or formulation may be coated onto plates, coated onto wire mesh, or indeed coated onto any solid support.

For example, the matrix may be formed as beads or other shaped particles, which may be retained on a support. A preferred support comprises a mesh or net, which may be made of metal, wire or other suitable material. The beads or particles may be formed by any suitable means, for example by spraying or misting from a nozzle onto the mesh, in a manner analogous to freeze drying. The matrix comprising the micro-organisms may be applied to the support by for example soaking the support (such as a wire mesh) in CaCl₂ (or solutions containing other divalent or alkaline earth metal ions as described elsewhere in this document) and subsequently immersing the mesh into alginate containing probiotic suspensions. The reverse is also possible, where the mesh is soaked in the probiotic suspension and then with calcium solution etc. Alternative means of coating are apparent to a skilled individual. The plates may then be aligned side by side in a holding vessel which may be supplied by medium. Effluent from the system would contain shed probiotic.

Furthermore, the matrix may be formed as a substantially extended surface, such as a substantially planar surface, and optionally supported by a support element. Thus, the matrix may be coated as a film onto a plate of suitable material, for example, an inert material such as metal or plastic. The support is preferably resilient. The support may comprise agar or agarose, preferably comprising a suitably high content of gelling agent for resilience. A preferred concentration is 1%. 2%, 3% or more of agarose or other gelling agent. An advantage of such a configuration is the increased surface area available for the micro-organisms to be detached. Thus, it will be understood that the surface may comprise indentations, ridges, or have a curved, sinusoidal, wavy or other cross sectional profile to increase the surface area.

Alternatively the immobilised probiotic could be plated onto the baffles of an impellor device which may be rotated through a medium at a particular speed (for example, at a low speed). The probiotic would then be shed into the medium. Such baffles may comprise the planar configurations described above.

The advantage of such configurations is especially evident in configurations where multiple plates (preferably arranged in substantially parallel form) are used.

While reference has been made to immobilising the micro-organism in a matrix, it will be appreciated that any form of immobilisation in conjunction with a suitable support material may be used, so long as the micro-organisms retain substantial viability and may be shed from the support or matrix. Thus, where we refer to immobilisation "within" or "in" a matrix, this should be taken to refer to any sort of immobilisation of micro-organism supported by a matrix. Included are immobilisation in, around, about, within, on the surface of, attached to, or otherwise associated with a matrix.

For example, the matrix may comprise a solid or semi-solid support, upon which the micro-organism is supported. Such a support may be any support, preferably an inert support, as known in the art. For example, the support may comprise a bead, upon which the micro-organism is attached. Various shapes are possible, for example, elongated, tubular, spherical, cubical, etc. The supporting material may comprise agar, agarose, carrigeenan, etc, as described above. Preferably, however, the micro-organism is embedded within the matrix, such that a substantial portion of matrix surrounds the micro-organisms. Embodiments in which the matrix acts as a substrate include those described in Example 3, where kelp is used as a support.

The matrix containing the micro-organisms may be freeze dried. Methods of freeze-dying substances are well known in the art. Alternatively, the matrix may be lyophilised by, for example, exposing to a vacuum, or spray dried. Various methods of lyophilisation and spray drying are known in the art. The matrix containing the micro-organisms may be reconstituted by adding water or medium (for example, broth or nutrient) to the dried matrix.

The micro-organism containing matrix may also contain a preservative which improves the viability of the micro-organisms. When micro-organisms are freeze-dried their reconstitution by addition of medium or water is enhanced by adding cryopreservants (cryoprotectants) prior to freezing. Preferably, the preservative is one which protects the micro-organisms from dehydration or cold, or both. The former includes agents which enable substantial survival or viability of micro-organisms, where water has been removed from the natural environment, preferably protecting them against complete dehydation.

Such preservatives and/or cryopreservants are normally characterised by the presence of numerous hydroxyl groups in their structure and it is believed that these groups stabilise micro-structure at a molecular level in the freeze-dried biological by replacing the hydroxyl groups of water removed during the freezing procedure. Agents such as glycerol, sucrose, glucose and more recently, trehalose have been shown to enhance rehydratability of biologicals. Trehalose is very efficient in this regard and has been exploited in preparing freeze-dried preparations of biologicals (Sanchez et al., 1999, Intl. J. Pharm. 185, 255-266; Esquisabel et al., 1997, J. Microencapsulation, 14, 627-638). In some cases crude preparations of preservatives have been employed to stabilise freeze-dried preparations of micro-organisms and it has been found that non-fat milk solids stabilise alginate-immobilised preparations of *Lactobacillus* strains (excluding *Lactobacillus acidophilus*) (Selmer-Olsen et al., 1999, J. Appl. Microbiol. 87, 429-437). Thus, for example, the preservative may comprise whey or whey permeate, or combinations of these. It is well known that milk may be separated into whey and curds; proteins such as residual caesin are removed from whey to produce whey permeate. Furthermore, any saccharide or polyalcohol may be used as a potential preservant.

The preservative or cryopreservant may be glycerol, sucrose, glucose, mannitol, sorbitol, adonitol, betaine (*N,N,N*-trimethylglycine), lactose or trehalose. In order to incorporate the cryopreservant into the matrix, the appropriate preservative or cryopreservant (for example, trehalose or lactose) is dissolved in the appropriate medium, preferably as a 2% w/v to 10% w/v lactose, more preferably 2%, 3%, 4% or 5%. The gelled alginate is then exposed to the preservative or cryopreservant solution for a suitable amount of time to allow the preservative or cryopreservant to enter the matrix. Alternatively, the beads are formed already comprising the preservative or cryopreservant. Thus, the preservative or cryopreservant may be added to the growing medium containing the micro-organism and alginate, before being gelled by exposure to calcium, etc. Other combinations are possible, and will be evident to the reader.

It will be appreciated that combinations of preservatives may be used. Thus, the matrix may comprise lactose and trehalose, or it may comprise whey, or whey permeate, together with lactose and/or trehalose, etc.

The micro-organisms which are suspended in the matrix may comprise a single strain or species. Alternatively and preferably, the matrix comprises more than one strain or species. For example, the matrix may contain *Lactobacillus acidophilus* LA-107 and *Enterococcus faecium* EF-101.

The formulation serves as an innoculative source of the probiotic. Micro-organisms may be detached from the formulation by mechanical action, for example, by directly contacting the formulation with the feed stream. Cells may be detached by being sloughed off, and are carried by the feed stream to the feeding point for the animal. The detached micro-organisms are suitably daughter cells arising from cell division of the suspended micro-organisms. Passage of feed particles in the feed stream may also wear away the alginate and release trapped micro-organisms. For example particles of the matrix may be dissolved in the feed stream where the feed stream is a liquid feed stream.

The formulation may be housed in a delivery device such as a cartridge. Housing of the formulation in a cartridge is advantageous when the formulation is in the form of alginate beads. Thus, the cartridge serves as a column to hold the beads. The beads may be packed into a cartridge for storage, and the cartridge retrieved for use. The cartridge has openings to allow passage of water past the beads. Preferably, the openings are formed as a mesh having a mesh size which is small enough to retain the beads in the cartridge, but large enough to allow detached cells to be carried away.

The immobilised probiotic formulation may be housed in a column as described above. This is advantageous as it enables a continuous or semi-continuous flow system to be set up, where the feed stream is passed through the column in a continuous or semi-continuous manner to allow regular discharge of micro-organisms. Such a continuous flow system is described in detail in Example 10. However, it will be appreciated that a batch or pulsed flow system may also be used.

Alternative configurations are also compatible with continuous or semi-continuous flow operation. These include the use of air-lift technology in which the immobilised probiotic preparation is housed within a vessel and air (or other gas, preferably an inert gas) is pumped in through the base of the system in order to facilitate agitation. Medium may be introduced through the bottom of the system and spent medium containing probiotic removed from the top of the system. The vessel may also advantageously be equipped with a gas vent, for example at the top or at any other suitable location.

Alternatively, a stirred tank system may be employed, in which the immobilised system is housed in a vessel and agitation accomplished using an impeller. Again fresh medium may be introduced to the system and spent medium containing probiotic harvested from the system.

Furthermore, the immobilised cells may be placed in a hollow fibre reactor and/or in the fibre-excluded space. The internal space of the fibres may be employed to supply the system with nutrient and to remove waste products and the excluded space may house the immobilised cells. The cells may be flushed with medium on a continuous basis to harvest cells shed from the immobilised system.

Continuous-flow semi-solid state configurations are also possible. Thus, the beads, matrix or other formulation comprising probiotic may be placed on a platform made of any suitable material (for example, a metal or plastic grid), above which moisture and nutrient are supplied as a spray. The microbes shed from the immobilisation matrix may then be harvested as the moisture drips from the grid and collected into a vessel placed beneath the grid. This system has the advantage of minimising the amount of liquid water in the system and provides an extremely high concentration of probiotic micro-organisms.

As can be seen from the above, where the beads, etc comprising the micro-organism are packed, for example, in the form of a column, it is advantageous to provide some form of agitation means in order to promote shedding of the micro-organism from the matrix. Any form of mechanical, electronic, magnetic or physical agitation may be used. Such agitation may be provided by use of a fluidised bed, as described in Example 10 below. Any suitable fluid such as air, water, medium etc may be pumped into the packed beads, etc comprising the micro-organism. The fluid may be passed via the beads etc continuously or preferably continuously. This enables mechanical action to detach the micro-organisms. The matrix may be set into motion (i.e., shaken) by means of the agitation fluid; alternatively, the matrix may be substantially immobile, and the detachment of the micro-organisms effected by passage of the agitation fluid over the fixed matrix.

The formulation may advantageously be placed directly in a liquid feed stream, for example, a supply of drinking water for the animal. Alternatively and preferably, the formulation may be positioned at a distance from the feed stream. In this case, the cells are detached by a separate mechanism and are conveyed (for example, by a pipe) to the feed stream after being detached. The detachment mechanism may comprise a flow of water or other liquid past the formulation, which detaches the micro-organisms from the matrix and carries them to the feed stream. The flow of liquid may be mediated by gravity. Preferably or in addition, the flow may be mediated or assisted by a pump.

Preferably and advantageously, the water or liquid feeding the delivery device comprises an appropriate nutrient broth or medium for the micro-organism. The nutrient broth or medium may be supplied from a reservoir, and the flow of broth or medium over the beads may be regulated to achieve an appropriate dose of micro-organism into the feed stream. Thus, when the beads are bathed in a flow of nutrient broth or medium, the immobilised cells are nourished and undergo cell growth and division, providing a supply of detached cells to be carried to the feed stream. In contrast, when the supply of nutrient broth is turned off, growth of the micro-organism population ceases.

The rate of detachment of micro-organism may thereby be adjusted so that it is substantially continuous and uniform to overcome the dosage and mixing problems associated with previous methods as described above. However, it may be preferred in some circumstances to have a pulsed, batch or semi-continous flow, and the apparatus is adapted to supply such a flow by methods known in the art.

The invention is further described, for the purposes of illustration only, in the following examples.

### EXAMPLES

### Example 1. Immobilisation of Probiotic Strains and Use as a Continuous Source of Viable Probiotic

The objective of this example is to demonstrate (1) that the microbial probiotic strains chosen can be immobilised without detrimental effects to viability and (2) that these formulations may be used as a continuous source of viable probiotic as a result of shedding from the immobilisation matrix.

100ml cultures of *Lactobacillus acidophilus* LA-107 (ATCC strain no. 53545) and *Enterococcus faecium* EF-101 (ATCC 19434) are grown to stationary phase in MRS (deMan, Rogosa. Sharpe) broth and nutrient broth (Oxoid), respectively at 37°C. Stationary phase is reached within 8 hours using EF-101 whereas the LA strain reaches stationary phase within 26h. Cells are washed in medium, recovered by centrifugation and suspended in 4ml (LA-07) and 2ml (EF-101) of 4% (w/v) sodium alginate. These suspensions are added drop-wise into 100ml of 50mM CaCl₂.

The resulting beads of the immobilised cultures (approximate diameter = 2-3mm) are retained at 4°C for 1 hour in the CaCl₂ and stored in either broth or simply as moistened beads at room temperature for one week. In order to determine whether or not the micro-organism in the matrix remains viable and capable of being shedded from the bead, a single bead is placed into 50ml of the relevant broth containing 50 millimolar CaCl₂, incubated at 37°C and samples of the broth are taken at the times indicated in Figure1A and Figure1B. If viable micro-organism is present, then shedding from the surface of the bead will contribute to turbidity in the surrounding medium.

The turbidity of these samples is determined by measuring the absorbance at 660nm using a spectrophotometer. The growth profiles obtained for the beads containing LA-107 are shown in Figure1A. Medium is replaced at 30 hours, 50 hours and 170 hours. The control sample consists of free cells which are re-fed at the indicated times by addition of fresh broth. The results demonstrate that cells in beads stored in medium for one week recover at about 10 hours and the growth profile is similar to that of the control, non-immobilised population of cells. In addition, when beads are re-fed at 30. 50 and 170h, growth is again recovered by shedding from the beads. Although beads stored for 1 week in the absence of medium fail to recover in the first cycle up to 30 hours, recovery is evident at subsequent refeeding cycles.

Growth profiles obtained following a similar study with the immobilised EF-101 strain are shown in Figure 1B. Refeeding is carried out at 7, 19, 26 and 32 hours. The results demonstrate that the recovery of the beads is similar to growth profiles exhibited using the free cells. In addition, it is found that when the bead is re-fed at 200 hours a similar recovery profile is obtained.

These results demonstrate that both of the probiotic micro-organisms listed above may be immobilised in alginate matrices and the micro-organism retains viability. In addition, the probiotic formulations retain their innoculative capability over a considerable period of time and innoculation from the formulations exhibit similar growth profiles to those exhibited by the free micro-organisms.

### Example 2. Immobilisation of Probiotic Strains by Growth in Medium Containing Soluble Alginate and Addition to Calcium

The above example describes the immobilisation of probiotic micro-organisms in matrices, by firstly harvesting probiotic microorganism following conventional growth on media, suspension in alginate, and drip-wise addition to CaCl₂ solutions.

This example shows the converse may also be used to immobilise the micro-organisms, i.e., growth of micro-organisms in medium comprising soluble alginate, and addition to calcium containing solutions. Microorganism (LA 107) is first grown in MRS medium containing 4% sodium alginate. These mixtures are then added dropwise to 50 mM CaCl₂ and the beads which formed are examined for their ability to shed probiotic micorganism into medium.

### Example 2 Results

When beads are prepared in the above alternative manner, they are placed in fresh MRS medium and the ability of the bead to shed microorganism is determined by measuring the absorbance at 660nm. In order to further demonstrate that shedding of microorganism can be carried out for a number of cycles, fresh medium is added at 37, 54 and 122 hours. The results obtained are shown in Figure 2 and they demonstrate that these formulations, prepared in the alternative manner above, may be employed to continuously yield probiotic microorganism.

### Example 3. Immobilisation of Probiotic Strains on the Surface of a Material (Kelp)

The above Examples show immobilisation of microorganism within a matrix. This Example demonstrates that probiotic micro-organisms may be immobilised by attachment to a solid or semi-solid material (substrate). Thus, as well as being immobilised within a matrix, the micro-organism may be immobilised on a material, which may itself be a matrix.

The microorganism is grown on blocks of kelp, which is obtained freshly from the sea-shore (Dunseverick, Co. Antrim, Northern Ireland). The inner pulp of the stem is cut into cubes measuring approx 125mm³ and washed thoroughly. These are added to flasks containing MRS medium, autoclaved, and following inoculation with LA 107, the flasks are placed in an orbital incubator at 37°C.

The cubes of kelp are found to be covered in a layer of microorganism, indicating that the micororganism had become immobilised onto the matrix. The liquid is decanted and the blocks of kelp together with the adhered microorganism are packed into a column. A similar configuration as that described in Example 10 (see below) was employed except that the flow rate is set at 85ml/hr., medium is introduced to the top of the column and the flow is facilitated by gravity. MRS medium is pumped through the packed bed of the column and viable microorganism is determined by direct counting on agar plates (MRS agar).

### Example 3 Results

The results obtained are shown in Figure 3 and they demonstrate that the formulation, based on immobilisation of the probiotic microorgansim onto a matrix is also capable of serving as a continuous source of probiotic. As in the case with the results shown in Figure 7A (see Example 10 below), output of microorganism from the column is erratic and this may be circumvented by agitation of the bed as described for Example 10. Nevertheless these results demonstrate that an alternative matrix may be employed in a column configuration for continuous inoculation of either animal drinking water or feed streams in order to facilitate inoculation of the digestive tract of recipient animals.

### Example 4. Long-Term Storage of a Probiotic Formulation Consisting of Enterococcus Faecium EF-101 Immobilised in Alginate

In order to determine whether or not immobilisation might provide advantage with respect to storage, *E. faecium* (EF-101) is chosen as a candidate probiotic micro-organism and immobilised as described for Example 1. The beads and free cells are then stored at 4°C in nutrient broth containing 5mM CaCl₂ for the times indicated in Table 1 and the medium is replaced on a weekly basis.

**Table 1 Stability of E. faecium EF-101 in the probiotic alginate formulation**

| | **Before encapsulation** | **1 week** | **1 month** | **3 month** |
|---|---|---|---|---|
| **Enterococcus strain** | | | | |
| Immobilised | 1.65 x10⁸ | 7.7 x10⁷ | 2.5 x10⁸ | 4.35 x10⁸ |
| free cell | | 6.15 x10⁷ | 2.3 x 10⁸ | 2.0 x10⁸ |

In order to determine viability of micro-organisms in the immobilisation matrix, a single bead is taken at the indicated times and dissolved in 50ml of nutrient broth containing 50mM NaCl and 10mM sodium citrate. Viable cell counts are determined following overnight growth by inoculation onto nutrient agar plates. The results in Table 1 demonstrate that viable micro-organism is retained in the beads for periods up to 3 months and viable counts are higher than those retained in preparations of the free cells.

The results demonstrate increased stability of the immobilised probiotic micro-organism over prolonged periods of time.

### Example 5. Inclusion of Lactose Into the Immobilised Probiotic Formulation Enhances Recovery of Viable Probiotic Micro-Organism Following Reconstitution of Freeze-Dried Preparations

This example determines whether or not the relevant probiotic strains can be immobilized in alginate, stored as a freeze-dried preparation (for enhanced shelf-life/storage manipulation) and reconstituted to yield viable micro-organism. We also wished to determine whether adding lactose to the beads prior to freeze-drying enhanced reconstitution of the immobilised preparation in terms of its inoculating capability. In particular, we wished to determine if lactose is as efficient as trehalose in enhancing such reconstitution.

In these studies LA-107 is chosen as the representative probiotic micro-organism and immobilised as described in Example 1. A preparation of 50 beads is suspended in 10ml of 2% (w/v) lactose or 2% (w/v) trehalose for 1 hour at room temperature prior to freeze-drying. Following freeze-drying the beads are stored at room temperature for 1 week. The control samples consist of non-immobilised, freeze-dried micro-organism which have been stored in medium for 1 hour prior to freeze-drying. After a week at room temperature single beads are rehydrated in 50ml of MRS medium containing 50 millimolar CaCl₂ and the inoculating capability of those preparations is examined as described in Example 1.

The results are shown in Figure 4. The inoculating capability of each immobilised preparation is compared with the inoculating capability of free, non-dried cells. The relative efficiency of the inoculating capability is indicated by the time taken for the medium to reach an absorbance of 0.5 at 660nm (A660) and this is reflective of the amount of microbial growth. It is found that inoculation by free cells exhibits a conventional profile and the A660 reaches 0.5 within 16h. The profile exhibited by freeze-dried free cells shows that an A660 of 0.5 is reached within 19h. Both the lactose and trehalose stabilised, immobilized and freeze-dried preparations reach an A660 of 0.5 within 21 hours and the profiles exhibited by both are very similar. The non-stabilised freeze-dried immobilized probiotic is slowest, and reaches an A660 of 0.5 at 34h.

The results clearly demonstrate the advantage of adding either lactose or trehalose to the immobilised formulation prior to freeze-drying. Since both inoculative profiles are similar the results suggest that lactose, rather than trehalose, is the preferred additive on the basis of formulation cost. The results also clearly demonstrate that the suggested probiotic formulation inclusive of lactose and/or trehalose may be stored as a dried product over prolonged periods of time.

### Example 6. Effect of Lactose and Trehalose on Long Term Recovery of Viability (6 month study)

The above Example shows that immobilising probiotic microorganism in alginate together with either trehalose or lactose provides a degree of protection when preparations are stored for a week following freeze-drying. In order to extend that study we decided to immobilise the same microorganism (LA 107) as described above and to continue this study to month 6.

In this case also preparations consist of microorganism stored in alginate alone, alginate plus 2% lactose and alginate plus 2% trehalose. In addition, immobilised micro-organisms are also stored in 4% lactose. Control samples consist of the free microorganism. Each preparation is stored for up to 6 months and samples are periodically re-constituted. Reconstituted microorganism preparations are then examined for their ability to shed probiotic microorganism into the surrounding medium for a period of 48 hours, and the absorbance at 660nm is determined using a spectophotometer. An increase in the absorbance is indicative of shedding of probiotic from the bead surface. No increase in absorbance is indicative of negative viability.

### Example 6 Results

The results obtained are shown in Tables 2A and 2B below.

**Table 2A Shedding of probiotic into medium following storage of freeze-dried immobilised preparations.**

| **Preparation** | **1Month** | **3Months** | **6Months** |
|---|---|---|---|
| **Absorbance at 660nm after growth for 30-45h** | | | |
| **Freeze dried (alginate)** | **0** | **0** | **0** |
| **Freeze dried control*** | **1.3** | **1.4** | **1.6** |
| **Freeze dried trehalose** | **1.4** | **1.3** | **1.4** |
| **Freeze dried lactose** | **1.3** | **0** | **0** |

| | | | |
|---|---|---|---|
| * *Freeze dried control consists of the freeze-dried non-immobilised microorganism.* | | | |

**Table 2B Effect of increasing concentration of lactose on storage of freeze-dried immobilised preparations of probiotic.**

| **Preparation** | **3 Months** | **6 Months** |
|---|---|---|
| **Absorbance at 660nm following growth at 30-48h** | | |
| **Freeze-dried control** | **1.4** | **1.6** |
| **Freeze-dried lactose (2%)** | **0** | **0** |
| **Freeze-dried lactose (4%)** | **1.6** | **1.4** |

| | | |
|---|---|---|
| * *Freeze dried control consists of the freeze-dried non-immobilised microorganism* | | |

As can be seen from Tables 2A and 2B, in the absence of any sugar additive, viability of the probiotic microorganism in alginate is not preserved at 1 month storage or later. When trehalose is employed as a stabiliser in the immobilised preparations viability is preserved for up to 6 months storage of freeze-dried preparations. However, when lactose is employed as a stabiliser at a concentration of 2% (w/v) in the immobilised preparations, viability of the probiotic is preserved for up to 1 month. However at longer storage times, viability of the probiotic in these lactose containing solutions could not be recovered (Tables 2A and 2B).

We thererefore decided to determine whether or not increasing the concentration of lactose might ensure survival of the probiotic in immobilised formulations. Therefore a similar experiment is performed in which viability of probiotic is examined in immobililsed formulations containing 4% (w/v) lactose and which are reconstituted following storage for 3 and 6 months. The results are shown in Table 2B and they demonstrate that 4% lactose preserves viability of probiotic in freeze-dried immobilised formulations for periods of up to 6 months.

Lactose may therefore serve to stabilise micro-organisms as an alternative to trehalose.

### Example 7. A Microbial Probiotic Formulation Consisting of Two Microbial Entities Co-Immobilised in An Alginate Matrix

In the above examples we employ both *Lactobacillus* and *Enterococcus* probiotic microbial strains immobilised separately in alginate matrix formulations. The objective in this study is to determine whether or not both the *Lactobacillus* and *Enterococcus* strains can be co-immobilised into the same matrix yielding a single formulation with inoculative capability for both probiotic micro-organisms.

In this study both LA101 and LA 107 are co-immobilised together with EF-101 to yield two separate formulations, namely LA-101/EF101 and LA-107/EF-101. Immobilisation is carried out as described in Example 1, except that the appropriate micro-organism mixture is employed instead of the single strain. In the LA-101/EF-101 mixture the ratio is 1:1 with respect to cell counts. In the case of the LA-107/EF-101 the mixture ration is 1:10. Following immobilisation, the formulations are stored for 4 days in medium. Control populations consist of the appropriate mixtures of micro-organism in similar ratios. After 4 days storage a single bead of each mixture is placed in MRS broth as described for examples above and the inoculating capability is determined be examining the growth of micro-organism shed into in the medium.

The results are shown in Figure 5 and demonstrate that micro-organism is released from both co-immobilised preparations. Although these results demonstrate that the co-immobilised preparations are capable of shedding micro-organism into the surrounding medium, it is necessary to examine whether or not both partner micro-organisms were being released from the matrix. To this end samples of broth are harvested from the co-immobilised systems and inoculated onto MRS agar plates (Oxoid) at a dilution of 1 in 10⁶. Colonies are distinguished on the basis of colony morphology and the relative proportion of *Lactobacillus* and *Enterococcus* is determined by direct counting. The results obtained are shown in Table 3 below.

In addition, each system is re-fed at 38 hours with fresh medium and grown for another cycle to stationary phase. Again, samples of broth are analysed as described above in order to determine the relative proportions of each partner micro-organism shed from the immobilisation matrix. The results obtained are also shown in Table 3 below.

**Table 3 Identification of microbial strains released from the co-immobilised formulations.**

| **Mixture** | **Cycle** | **Strain EF** | **Strain LA** |
|---|---|---|---|
| **LA-101/EF-101** | **1** | **52** | **16** |
| **LA-107/EF-101** | **1** | **76** | **9** |
| **LA-101/EF-101** | **2** | **15** | **38** |
| **LA-107JEF-101** | **2** | **0*** | **33** |

Counts determined by inoculating 1 in 10⁶ dilutions of cultures onto MRS agar plates and colonies distinguished on the basis of morphology. * The absence of colonies means that at this dilution no EF could be detected it does not necessarily mean an absence of EF in the medium.

The results demonstrate that at the end of the first growth cycle the EF-101 strain is the predominant strain released from the matrix into the medium. However, the results also demonstrate the presence of LA-101 and LA-107 released from the relevant immobilised preparation. This is to be expected since growth of the EF-101 strain is much faster than both of the LA strains, as described above in Example 1. There was some concern that the EF strain might out-compete the LA strains in the co-immobilized preparation, resulting in eventual disappearance of the latter in terms of inoculative capability. However, when the mixed formulations are re-fed and again analysed for the relevant amounts of EF and LA it is surprisingly found that the latter becomes the predominant micro-organism shed into the medium (see Table 3).

The results demonstrate that both probiotic microbial strains may be co-immobilised into alginates to produce a novel probiotic formulation and that formulation may be employed to facilitate inoculation of both partner strains of the micro-organism into the relevant medium. In addition, the results demonstrate that it is possible to achieve selective output from the beads by manipulating the relative proportions of each partner during formulation. This would provide advantages in systems where it is desired to preferentially supply one of a pair of microorganisms to a host prior to supplying the other micro-organism of the pair.

### Separate Beads

The results above demonstrate that it is possible to obtain mixed cultures of probiotic from the immobilised formulations when two probiotic micro-organisms are co-immobilised into a single bead of the matrix. An alternative to such a system involves mixing two separate beads containing each microorgansim. To this end beads are prepared containing either LA 107 or EF as described in Example 1 above. Beads are then washed and harvested and then two beads, one containing LA 107 and the other containing EF are placed in MRS medium. Viable counts are determined at mid-log phase and medium is subsequently replaced with fresh medium so that 3 shedding cycles are examined.

The results obtained are shown in Table 4 below.

**Table 4 Combination of beads 1xLA107 and 1xEF 101. TMTC = Too Many To Count**

| **Shedding Number** | **Counts/ml @ mid log** |
|---|---|
| | |
| **Shedding 1** | |
| **LA107** | **0** |
| **EF** | **TMTC (at dilution of 10⁻⁴)** |
| | |
| **Shedding 2** | |
| **LA107** | **24x10³** |
| **EF** | **0.8x10³** |
| | |
| **Shedding 3** | |
| **LA107** | **54.7x10⁵** |
| **EF** | **0** |

The results shown in Table 4 demonstrate that both micororganisms are shed into the medium (Shedding 2). The results also confirm those obtained with both micro-organisms in a single bead (above) where the *Lactobacillus* strain eventually becomes predominant in the medium at the third shedding. These results confirm that more than one probiotic microorganism may be employed in formulations and these may either be co-immobilised into the matrix and/or immobilised into separate beads.

### Example 8. Pre-Biotics

It has been shown in the above Examples that probiotic micro-organisms may be incorporated into matrices and that these formulations provide viable probiotic microorganism which may either be administered directly to the animal or introduced to either drinking water or feed streams. In order to further illustrate advantages associated with these formulations, we determined whether other materials might be co-immobilised together with the probiotic microorganism.

It has been known for some time that certain non-digestible oligosaccharides may provide advantage to probiotics by either stimulating growth or function of the probiotic cultures in the digestive tract and these oligosaccharides are commonly referred to as prebiotics (Crittenden et al., 2000, J. Appl. Microbiol., 90, 268-278). It was felt that if these could be co-immobilised together with the probiotic in the immobilising matrix then these could be co-administered to the recipient animal thereby providing a prebiotic and probiotic function in a single dose. We therefore decided to choose a series of non-digestible carbohydrate entities and co-immobilise these together with the probiotic microorganism LA 107.

To this end the microorganism is grown in MRS medium and harvested. This is then added to 4% (w/v) alginate containing 2% (w/v) prebiotic and the candidates chosen are inulin, cellulose, resistant starch and oat spelt xylan. Beads are formed as described in Example I above and these are then placed in medium. The ability of the microorganism to shed into the surrounding medium is examined. The medium is replaced at 29, 50 and 121 hours in order to demonstrate prolonged shedding.

In addition, the preparations are also stored as moist preparations for a 1 month period at room temperature, 4°C and at -20°C, and the ability to recover viable microorganism after placing in medium examined at the end of this period. In these particular studies the microorganism is grown in either prebiotic plus medium and then immobilised in alginate, or the microorganism is grown in medium and then immobilised in alginate containing the prebiotic entity. Control preparations used throughout the studies described in this example consist of probiotic microorganism immobilised in alginate alone.

### Example 8 Results

In order to determine whether or not the probiotic microorganism can be shed from the formulations containing the prebiotic, beads are placed in medium and the absorbance at 660nm is determined. Shedding cycles are examined after transfer of the beads to medium at 0, 29, 50 and 121 hours. The results are shown in Figure 6 and they demonstrate that the microorganism is shed from all formulations examined. These results demonstrated that additives such as prebiotics which provide benefit may be incorporated into the immobilisation matrix without adversely effecting the ability of those formulations to provide probiotic microorganism.

In addition to the above results, we also decided to determine whether or not the incorporation of the prebiotic into the formulation would provide benefit in terms of storage. To this end microorganism is prepared by either growth in the presence or absence of prebiotic and then immobilised in alginate. In the latter case, prebiotic is added to the alginate after immobilisation.

In this case also the prebiotics chosen are inulin, cellulose, resistant starch or oat spelt xylan. Beads were formed and stored as indicated in Table 5 below. Following storage for one month the beads are added into MRS medium and the viable cell count is determined at late stationary phase. The results are shown in Table 5.

**Table 5 One month storage results of LA-107 with various prebiotics.**

| **Sample** | **V.C.C before immobili sation** | **4°C** | **R.T.** | **-20°C** |
|---|---|---|---|---|
| Alginate only | 1.2x10⁸ | no count | no count | no count |
| *Inulin + MRS | 1.8x10⁸ | no cell count | no cell count | 2.2x10⁸ |
| +Inulin + Alginate | 1.8x10⁸ | 4.8x10⁸ | no cell count | 5.9x10⁸ |
| Cellulose + MRS | 2.8x10⁸ | no cell count | 3.8x10⁸ | 6.6x10⁸ |
| Cellulose + Alginate | 1.8x10⁸ | 5.6x10⁴ | no cell count | no cell count |
| Resistant starch + MRS | 2.9x10⁸ | 6.9x10³ | 1.5x10⁸ | 5.4x10⁸ |
| Resistant starch + Alginate | 2.2x10⁸ | 5.5x10³ | 2.2x10⁸ | 4.2X10⁷ |
| Oat spelt xylan + MRS | 3.6x10⁸ | 5.2x10⁸ | no cell count | 1.5x10⁷ |
| Oat spelt xylan + Alginate | 2.3x10⁸ | 4.5x10⁸ | 2.4x10⁸ | 4.2x10⁸ |

| | | | | |
|---|---|---|---|---|
| * *Grown together with prebiotic and then immobilised + Grown in MRS and then immobilised in alginate plus prebiotic.* | | | | |

Table 5 shows that after one month storage under all conditions no viable counts are recovered from the formulation which consisted of alginate and probiotic alone.

In the case of both resistant starch and oat spelt xylan counts are recovered even when formulations are stored at room temperature. Although no counts are recovered from formulations containing inulin following storage at room temperature, counts are recovered from formulations stored for one month at either 4°C and at - 20°C. In the case of cellulose-containing formulations a similar pattern emerged although in this case and depending on how the microorganism was prepared, wefound that counts could be recovered from formulations stored at room temperature. These results demonstrate that the addition of prebiotic to the immobilised formulations provide advantage in terms of storage. Where the prebiotic is also supplied to an animal (for example, as a supplement to feed), this may serve to improve stability of the micro-organism in the gut of the animal.

### Example 9. Immobilisation of Micro-organisms Increases Adhesion Capability

It is well documented that probiotic micro-organisms adhere to the lining of the digestive tract. Furthermore, it has been suggested that this property is important in mediating the beneficial effects of those probiotics (Tuomola et al., 2001, Am. J. Clin. Nutr. 73, 393S-3985; Kankaanpaa et al., 2001, FEMS Microbiol. Lett, 194, 149-153). In some cases it has been suggested that this property is functional in retaining the microorganism in the digestive tract while it exerts its beneficial effects. In other cases it has been suggested that other probiotic strains compete with enterotoxigenic microoganisms for adhesion sites, thereby out-competing the latter (Jin et al., 2000. Appl. Environ. Microbiol., 66, 42004204).

Since this phenomenon seems to influence the effectiveness of any probiotic, and, in particular, relates to the degree to which the digestive tract of a recipient would be colonised by that probiotic, we wished to determine whether or not the formulations described here might have any beneficial effects in terms of enhanced adhesion characteristics.

We harvested preparations of free microorganism, as well as microorganism derived from a freshly prepared immobilised probiotic formulation (Example 1 above), microorganism derived from immobilised probiotic formulation which has been freeze-dried (Example 5 above) and microorganism derived from a column as described in Example 10 below. These preparations of microorganism are then placed in contact with target mammalian cells and the degree to which they adhere to the cells is determined.

Essentially the method involves using HeLa cell in an adhesion assay. The cell line (ECACC no: 93021013) is obtained from the European Collection of Animal Cell Cultures. Cells are cultured in Eagle's minimum essential medium (MEM) (Life Technologies, Paisley, Scotland), supplemented with 10% foetal bovine serum (FBS) (Life Technologies) and 1% non-essential amino acids (Life Technologies). Cells are cultured in a humidified 5% CO₂ atmosphere at 37°C. A four-well chamber slide (Life Technologies) is used in each adhesion assay. Cells are prepared in monolayers in the chamber slide at a concentration of 1x10⁵ cells per well (1.7 cm² culture area per well) for 24 hours before performing the adhesion assay. Prior to the adhesion assay, the LA 107 bacterial culture is harvested by centrifugation at 3,000 g for 20 minutes. The bacterial pellet is then resuspended in MEM without FBS and non-essential amino acids. The colony forming units per ml. (CFU/ml) is determined by plating serial 10-fold dilutions of bacterial suspensions on MRS agar prior to the adhesion assay. The bacterial suspension of LA107 in MEM is 10-fold serially diluted in MEM to a concentration of 10⁻³. The adhesion assay is performed by firstly removing the medium from the HeLa cells and rinsing once with MEM. 1ml aliquots of bacterial suspension in MEM at the 10-3 concentration are added to each of three wells. The fourth well is used as a control and involves addition of medium alone. The chamber slide is then incubated at 37°C for a maximum of two hours. The monolayers are then washed twice with sterile PBS, fixed and stained using the Diff-Quik Stain Set (Gamidor Limited, Oxfordshire, England) and then examined microscopically. A visual counting method is used to determine the number of bacterial cells adhered to 100 target HeLa cells through random analysis of 100 cells per chamber. An average of three chamber lanes is assessed and the experiment is performed in duplicate. An average value is determined and multiplied by the dilution factor of bacterial cells used. In all tested storage conditions of LA107, a dilution factor of 10⁻³ is found to be the highest amount possible to accurately manually count the bacteria. In all cases viable counts are taken before the adhesion assay and compared against adhesion assay results.

### Example 9 Results

The results are shown in Table 6 below.

**Table 6 Adhesion Properties of Various Preps**

| | |
|---|---|
| **TEST SAMPLE (LA107)** | ***CELLS ADHERED/10⁶** |
| **NON-IMMOBILISED** | **1.63X10³** |
| **IMMOBILISED** | **4.71X10³** |
| **IMMOBILISED (FD)** | **11.8X10³** |
| **COLUMN** | **29.6X10³** |

| | |
|---|---|
| *Represents the number of bacterial cells adhered to 100 target HeLa cells following contact with 10⁶ cells of the test sample | |

In all cases the numbers of cells adhered are representative of those bound to the target cell per 10⁶ microbial probiotic cells added to the assay. The results demonstrate that all immobilised preparations of probiotic yield microorganism which exhibited a higher degree of adhesion than that exhibited by the free probiotic. Of the microorganism derived from the immobilised formulations, the probiotic derived from the column exhibits the highest degree of adhesion. The results suggest that immobilised probiotic formulations provide advantage in terms of yielding probiotic with superior adhesion characteristics.

### Example 10. A Continuous Flow Reactor System as a Source of Probiotic Micro-Organisms

An advantageous use of the immobilised probiotic formulations described in this document is to facilitate the continuous supply of viable probiotic microorganism to a drinking water system. We therefore decided to place the formulation in a continuous flow reactor system and determine whether or not the system is capable of serving as a continuous source of viable probiotic.

To this end it was decided to immobilise the probiotic microorganism LA107 as the candidate microorganism as described for Example 1 above and to pack the beads into a water-jacketed glass column (internal volume = 3cm x 30cm and the bead bed volume was approximately 106cm³). Beads packed into the column are retained using wire gauze at the top in order to ensure that the system is operated under packed-bed conditions. The column is maintained at 37°C using the water jacket and is supplied with MRS medium through the bottom of the column. Spent medium containing microorganism is removed from the top of the column and harvested at various times in order to determine the viable cell counts. In addition, the column is fitted with a filtered gas vent in order to ensure equalised pressure. The flow of medium is maintained at approximately 150ml/hr using peristaltic pumps.

In an alternative configuration, a circulating pump is attached to a closed circulation loop on the column such that the column is operated in a fluidised bed mode. Here, the beads are free to move. Again, this column is operated at a flow rate of 150ml/hr.

### Example 10 Results

The results obtained from these experiments are shown in Figures 7A and 7B. The results shown in Figure 7A represent those obtained from the column run in a packed bed operating mode.

When viable cell counts are determined during operation of the column over a prolonged period of time (up to 30 days), it is found that the output from the column is somewhat erratic. We also noticed that when the column matrix (beads) is examined in the column, microorganism had accumulated to such a degree that it became visually obvious as a build-up within the column. The high points shown in Figure 7A result from parts of this build-up periodically breaking away from the beads and being flushed from the column.

Although output from the column is erratic these experiments demonstrate that the column containing the immobilised probiotic serves as a continuous supply of probiotic. Furthermore, we demonstrate that the system can be operated for prolonged periods of time. Thus, this system is capable of serving as a source of probiotic for inoculation into a drinking water system and/or for inoculation of probiotic onto or into solid feed preparations.

In order to determine whether or not the erratic output of probiotic microorganism from the system can be adjusted in order to facilitate a more predictable output, we decided to run the column under fluidised bed conditions. This was facilitated by linking a closed-loop circulation system to the existing column configuration, such that the bed of beads become fluidised. When probiotic output is examined from this system we found that an even output is facilitated (Figure 7B). This demonstrates that any combination of configurations may be employed to facilitate any desired output from the column and again demonstrates that the system can serve as a continuous source of probiotic for inoculation of either drinking water and/or food preparations.

### Example 11. A Drinking Water Configuration Based on the Use of the Immobilised Microbial Probiotic Formulation(s)

Micro-organisms which are immobilised may be supplied to the feed stream, for example, using an apparatus as described below with reference to Figure 8.

The apparatus consists of a main water feed line (4) exiting from a primary water source (either mains or storage tank, 1). The main water feed line terminates at drinking outlets (5) from which the animals receive water. Water in this system may be maintained at the ambient room temperature in the animal housing facility. A cartridge containing the immobilised formulation (3) is attached to the main feed line (4) between the water source and the drinking outlets (5). Supply of microbial probiotic from this system may be mediated by gravity and/or by pump (6). The construction of the cartridge includes a mesh system such that the immobilised microbial probiotic is retained within the system but free microbial probiotic will exit from the system into the drinking water feed line. The cartridge may also incorporate some form of temperature control facility.

A pump (6) may be necessary where semi-continuous inoculation of microbial probiotic into the drinking water system is required, and is advantageous when more precise control of the amount of microbial probiotic in the drinking water is needed. However, and as mentioned above, the free microbial probiotic may be fed into the main feed line by gravity flow.

An additional nutrient re-feed cartridge (2) may also be provided containing nutrients required to facilitate maximum viability within the probiotic cartridge (3). The overall configuration is designed to deliver the optimal probiotic dose to the recipient animal.

In an alternative embodiment, the cartridge may be "inline" with the water flow from the mains water source to the drinking outlet. Thus, the cartridge may have an inlet allowing inflow of water from the water source, and an outlet allowing exit of micro-organisms, carried by the water stream. This alternative is shown as (4A) in Figure 8. A separate water source may be used in this embodiment, as described below.

It will be appreciated that the main or mains water source may contain components which may be toxic to the micro-organism, or which inhibit its growth (for example, chlorinated or fluoridated water). Therefore, in a further embodiment, a separate water source (of purified water or water which is biologically more compatible with the micro-organism) may feed the probiotic cartridge (3) - whether inline or not - to entrain the micro-organisms.

### Example 12. Evaluation of a Probiotic Delivered Via the Drinking System in Young Broilers

This Example describes a protocol complying with the GCP(V) standard to test the effect of a probiotic administered via the drinking water on the gut microflora and bird performance of young growing broilers. The particular example relates to testing of Lactobacillus species, but may be adapted as necessary for any probiotic microorganism.

Two treatments are used, the first as a control with 0% dosage of probiotic, and the second with a supplement of 2.5% probiotic.

The immobilised probiotic is supplied as beads of approximately 1-4 mm in size. Prior to addition to drinking water, one bead is incubated overnight to 48 hours at 37°C in 100 ml aliquots of MRS broth. 25 ml of this is made up to 1 litre with drinking water.

Only de-ionised or filtered water is used. The test substance is supplied to the animals in clean drinking water, in bottles of 1 litre volume, which are changed at 24 hourly intervals.

Details of lot numbers, expiry dates and volume/mass of test substances received and used are recorded.

Typical UK female broiler chicks are used (n = 60) as test animals, either Cobb 500 or Ross 308 broiler chicks depending upon availability. The strain used and the source of the chicks are documented in the raw data for the study, as are details of vaccinations the chicks are given whilst at the hatchery.

The trial is conducted as a non-blinded two treatment study where chicks are housed in groups of three in cages from day old to 28 days of age. Ten cages are used per treatment group, giving a total of 30 chicks per treatment and 60 chicks for the trial in total. This experimental design means that each cage is the replicate for analysis purposes and has the advantage over single chick housing in that if any mortalities occur in a replicate then the whole replicate is not lost. Thus the trial remains balanced and allows for robust statistical appraisal of the data.

A randomised-block design is used where chicks are randomly assigned to cages, and cages are randomly assigned to treatment group. The data, where appropriate, is analysed using analysis of variance, with suitable transformation where necessary (Minitab Release 7.2).

### Husbandry/Environment

The current *Codes of Recommendations for the Welfare of Livestock - Turkeys*/*broilers*/*layers* are adhered to, to cover aspects of the environment such as levels of carbon dioxide and ammonia, humidity, temperature, etc. These codes are available from the Animal Welfare Division, The Ministry of Agriculture, Fisheries and Food, Government Buildings (Toby Jug Site), Hook Rise South, Tolworth, Surbiton, Surrey KT6 7NF. Reference is made in particular to publications PB0077 (1987, 1993 reprint). PB0076 (1987), PB1315 (1994). PB1739 (1994), which are also available at
http://wwv.maff.gov.uk/animalh/welfare/publications/booklets/pb0076 fowlcode.htm
http://www.maff.gov.uk/animalh/welfare/publications/layhen-pub.htm and
http://www.maff.gov.uk/animalh/welfare/publications/booklets/pb0077/turkcode.htm

Bird Placement: Chicks are day old at the start of the study and are whole-room brooded using gas brooders, the brooders being adjusted to provide a temperature of 32°C. The brooder temperatures are decreased by 0.5°C until a temperature of 21°C by 31 days of age is achieved.

Feeder Space: Each cage has a feeding trough at the front where chicks will have free access to their feed.

Drinker Space: An individually labelled drinker bottle (volume 1 L) is positioned at the front of each cage.
Lighting: Day-old to 28 d lighting are 23h light and 1h dark.
Diet and Nutrition
Diet: The birds are fed normal commercial broiler diets, formulated to be adequate with regard to energy and nutrients for broilers of the sex, age and strain used in the study. The birds are fed ad lib using a diet and feeding programme that follows the recommendations of the breeding company specific to the strain of broiler used in the trial. This comprises a grower ration, in mash form, which are fed from 0-28 days of age. The estimated nutrient composition of the diet is recorded and included as study raw data.

Typical commercial broiler diets include an anti-coccidiosis drug, a prophylactic antibiotic, and a feed enzyme is often included. For the purposes of this study, no antibiotic, the feed enzyme or coccidiostat is included in the diet Feed Requirements: Each broiler consumes 2.29 kg by 28 days of age. 140 kg of feed is therefore required for the whole study.

Sampling of Feed: Feed samples are taken of the diet in triplicate and retained for *post hoc* subsequent analysis, if required. The feed samples are retained in a freezer (-18 ± 2 °C) for six months after acceptance of the final report, at which point they are discarded.

### Record Keeping

Data Recording: All data is recorded on standard ASRC data capture forms.

Body Weight: Each bird is weighed at the start of the experiment (day 0) and again at 7, 14, 21 and 28 days of age. Birds are weighed individually to the nearest 1.0 g.

Feed: Daily feed allocations are recorded and a feed weigh-back are carried out at 7, 14, 21 and 28 days of age. The difference between cumulative daily feed allocations and the feed weigh-back for that period, eg 0-7 d, are taken as the feed intake for that period.

Water Consumption: Daily water consumption are recorded (by weight) and summed for each weekly period.

Temperature and Relative Humidity: All maximum/minimum thermometers are read and recorded daily. Daily relative humidity will also be calculated and recorded.

Mortality: All deaths are recorded and where possible a cause are assigned on the basis of external inspection. *Post mortems* will only be routinely carried out in the event of a suspected disease scenario or if cause of death is unknown.

Faecal Samples: Faecal samples are taken from each cage (pooled therefore for all chicks within a cage) on a weekly basis. Faecal samples are analysed on a fresh basis for *lactobacillus* at a microbiology laboratory. Cloacal Swabs: Cloacal swabs are taken from a single bird from each cage on a weekly basis. These are analysed on a fresh basis for *lactobacillus* at a microbiology laboratory.

Microbiological Analysis of Faecal and Cloacal Samples: All faecal and cloacal samples are analysed on site for a total *lactobacillus* count on MRS media supplemented with the following selective antibiotics: 5 µg/ml Kanamycin, 500 µg/ml nalidixic acid and 75 µg/ml of laxicin.

Tissue Samples: At the end of the trial, all the chicks are humanely sacrificed by administration of an overdose of anaesthetic and whole GIT tract samples are collected and stored frozen for analysis.

Further analysis is conducted by the following protocols.

### Confirmation of LA107 from GIT tract samples by randomly amplified polymorphic DNA (RAPD) fragments

The RAPD analysis uses a single 10-mer primer to obtain a reproducible and unique fingerprint profile for the bacterial strain. PCR amplification is performed using the single primer of arbitrary nucleotide sequence: 5' AGCAGCGTGG 3', according to the protocol of Cocconcelli et al (1995) Development of RAPD protocol for typing of strains of lactic acid bacteria and enterococci. Letters Appl. Microbiol. 21:376-379.. Prior to analysis of GIT tract samples an RAPD profile of LA107 is generated.

### Identification of Lactobacillus using API 50 CH strips and API 50 CHL medium

API 50 Ch strips allow the study of the carbohydrate metabolism of micro-organisms. The API 50 CHL medium, which is used to inoculate the strips is intended for the identification of the genus Lactobacillus and realted organisms. This medium enables the fermentation of 49 carbohydrates on the API 50 CH strip to be studied (bioMerieux, Lyon. France).

On performing the above study, we find that micro-organisms which are suspended in the beads are delivered effectively to the animals. We also find that the delivered micro-organisms colonise the gut of the animals.

Each of the applications and patents mentioned above, and each document cited or referenced in each of the foregoing applications and patents, including during the prosecution of each of the foregoing applications and patents ("application cited documents") and any manufacturer's instructions or catalogues for any products cited or mentioned in each of the foregoing applications and patents and in any of the application cited documents, are hereby incorporated herein by reference. Furthermore, all documents cited in this text, and all documents cited or referenced in documents cited in this text, and any manufacturer's instructions or catalogues for any products cited or mentioned in this text, are hereby incorporated herein by reference.

Various modifications and variations of the described methods and system of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

**ANNEX I. LIST OF MICRO-ORGANISMS (FROM COUNCIL DIRECTIVE 70/524/etc)**

| **N°** | **Additive** |
|---|---|
| E 1700 | *Bacillus licheniformis* (DSM 5749), Bacillus subtilis (DSM 5750) (In a 1/1 ratio) |
| 1 | *Bacillus cereus* var. *toyoi* NCIMB 40112/CNCM I-1012 |
| 3 | *Saccharomyces cerevisiae* NCYC Sc 47 |
| 4 | *Bacillus cereus* ATCC 14893 |
| 5 | *Saccharomyces cerevisiae* CBS 493.94 |
| 6 | *Saccharomyces cerevisiae* CNCM I-1079 |
| 7 | *Saccharomyces cerevisiae* CNCM I-1077 |
| 8 | *Enterococcus faecium* ATCC 53519. *Enterococcus faecium* ATCC 55593 (In a 1/1 ratio) |
| 9 | *Pediococcus acidilactici* CNCM MA 18/5M |
| 10 | *Enterococcus faecium* NCIMB 10415 |
| 11 | *Enterococcus faecium* DSM 5464 |
| 12 | *Lactobacillus farciminis* CNCM MA 67/4R |
| 13 | *Enteroccocus faecium* DSM 10 663/ NCIMB 10 415 |
| 14 | *Saccharomyces cerevisiae* MUCL 39 885 |
| 15 | *Enteroccocus faecium* NCIMB 11181 |
| 16 | *Enteroccocus faecium* DSM 7134, *Lactobacillus rhamnosus* DSM 7133 |
| 17 | *Lactobacillus casei* NCIMB 30096 |
| | *Enterococcus faecium* NCIMB 30098 |
| 18 | *Enterococcus faecium* CECT 4515 |
| 19 | *Strepotococcus infantarius* CNCM I-841 |
| | *Lactobacillus plantarum* CNCM I-840 |
| 20 | *Bacillus lecheniformis* (DSM 5749), *Bacillus subtilis* (DSM 5750) (In a 1/1 ratio) |

## Claims

1. A method of delivering a micro-organism to a feed stream for subsequent ingestion by an animal, the method comprising:
(a) providing a formulation comprising a micro-organism suspended in or on a matrix;
(b) providing a feed stream for the animal;
(c) providing a flow of water or other liquid for the formulation, wherein the feed stream and the flow of water or other liquid may be the same stream, or the flow of water or other liquid may be positioned at a distance from the feed stream:
(d) detaching micro-organisms from the matrix, and entraining the detached micro-organisms into the flow of water or other liquid; and
(e) enabling an animal to ingest the feed stream and the flow of water or other liquid.

2. An apparatus for delivering a micro-organism to a feed stream for subsequent ingestion by an animal, the apparatus comprising:
(a) a formulation comprising a micro-organism suspended in or on a matrix;
(b) means for providing a feed stream for the animal;
(c) means for providing a flow of water or other liquid for the formulation, wherein the feed stream and the flow of water or other liquid may be the same stream, or the flow of water or other liquid may be positioned at a distance from the feed stream;
(d) means for detaching micro-organisms from the matrix; and means for entraining the detached micro-organisms into the flow of water or other liquid; and
(e) means for enabling an animal to ingest the feed stream and the flow of water or other liquid.

3. A method or apparatus according to Claim 1 or 2, in which the flow of water or other liquid is positioned at a distance from the feed stream, and further comprising conveying means for conveying detached micro-organisms to the feed stream.

4. An apparatus according to Claims 2 or 3, further comprising means for adjusting the flow of water or other liquid past the formulation.

5. An apparatus according to Claims 2-4, wherein the flow of water or other liquid comprises a nutrient broth.

6. A method or apparatus according to any preceding claim, in which the micro-organisms are detached from the formulation by the flow of water or other liquid past the formulation.

7. A method or apparatus according to Claims 1 or 2, in which the micro-organisms are detached by contacting the formulation with the feed stream.

8. A method or apparatus according to Claims 1, 2, or 7, in which the feed stream is a liquid feed stream.

9. A method or apparatus according to any preceding claim, in which the micro-organisms are detached from the matrix in a pulsed or a substantially continuous manner.

10. A method or apparatus according to any preceding claim, in which the feed stream comprises drinking water for the animal.

11. A method or apparatus according to any preceding claim, in which the feed stream and/or the flow of water or other liquid is delivered continuously or semi-contiuously.

12. A method or apparatus according to any preceding claim, in which the micro-organism is a probiotic organism, including a recombinant probiotic organism.

13. A method or apparatus according to any preceding claim, in which the formulation further comprises a prebiotic.

14. A method or apparatus according to Claim 13 in which the prebiotic is selected from the group consisting of: non-digestible oligosaccharide, inulin, cellulose, resistant starch, and oat spelt xylan.

15. A method or apparatus according to Claim 13 or 14, in which the prebiotic is present at a concentration of about 2%(w/v).

16. A method or apparatus according to any preceding claim, in which the micro-organism is a *Lactobacillus* or an *Enterococcus,*

17. A method or apparatus according to any preceding claim, in which the micro-organism is selected from *L. acidophilus* LA-107 (ATCC #53545), *L. acidophilus* LA-101 (ATCG #4356), and *E. faecium* EF-101 (ATCC # 19434).

18. A method or apparatus according to any preceding claim, in which the formulation comprises more than one strain or species of micro-organism.

19. A method or apparatus according to any preceding claim, in which the matrix comprises a water porous material.

20. A method or apparatus according to any preceding claim, in which the matrix comprises alginate, preferably calcium alginate or barium alginate.

21. A method or apparatus according to any preceding claim, in which the formulation is substantially free of water.

22. A method or apparatus according to any preceding claim, in which the matrix has been subjected to freeze-drying, spray drying or lyophilisation.

23. A method or apparatus according to any preceding claim, in which the matrix comprises a preservative or cryopreservant to maintain the viability of the micro-organisms.

24. A method or apparatus according to Claim 23, in which the preservative or cryopreservant comprises trehalose or lactose, or a combination of trehalose and lactose.

25. A method or apparatus according to Claim 23 or 24, in which the preservative or cryopreservant comprises lactose.

26. A method or apparatus according to an preceding claim, in which the formulation is provided in a delivery device.

27. A method or apparatus according to Claim 26, in which the delivery device is a cartridge.

28. A method or apparatus according to Claim 27, in which the cartridge comprises a mesh to retain the formulation in the form of beads within the cartridge, but substantially enabling detached micro-organisms to be carried away with the feed stream.

## Patentansprüche

1. Verfahren zur Zuführung eines Mikroorganismus an einen Futterstrom zur anschließenden Aufnahme durch ein Tier, wobei das Verfahren Folgendes umfasst:
(a) Bereitstellen einer Formulierung, die einen in oder auf einer Matrix suspendierten Mikroorganismus umfasst;
(b) Bereitstellen eines Futterstroms für das Tier;
(c) Bereitstellen eines Stroms von Wasser oder einer anderen Flüssigkeit für die Formulierung, worin der Futterstrom und der Strom von Wasser oder einer anderen Flüssigkeit den gleichen Strom darstellen können, oder der Strom von Wasser oder einer anderen Flüssigkeit vom Futtersttrom entfernt positioniert sein kann;
(d) Loslösen der Mikroorganismen von der Matrix, und Mitführen der losgelösten Mikoorganismen im Strom von Wasser oder einer anderen Flüssigkeit; und
(e) Befähigen eines Tiers zur Aufnahme des Futterstroms und des Stroms von Wasser oder einer anderen Flüssigkeit.

2. Vorrichtung zur Zuführung eines Mikroorganismus an einen Futterstrom zur anschließenden Aufnahme durch ein Tier, wobei die Vorrichtung Folgendes umfasst:
(a) Eine Formulierung, die einen in oder auf einer Matrix suspendierten Mikroorganismus umfasst;
(b) Mittel zum Bereitstellen eines Futterstroms für das Tier;
(c) Mittel zum Bereitstellen eines Stroms von Wasser oder einer anderen Flüssigkeit für die Formulierung, worin der Futterstrom und der Strom von Wasser oder einer anderen Flüssigkeit den gleichen Strom darstellen können, oder der Strom von Wasser oder einer anderen Flüssigkeit vom Futterstrom entfernt positioniert sein kann;
(d) Mittel zum Loslösen von Mikroorganismen von der Matrix; und Mittel zum Mitführen der losgelösten Mikroorganismen im Strom von Wasser oder einer anderen Flüssigkeit; and
(e) Mittel zum Befähigen eines Tiers zur Aufnahme des Futterstroms und des Stroms von Wasser oder einer anderen Flüssigkeit.

3. Verfahren oder Vorrichtung nach Anspruch 1 oder 2, worin der Strom von Wasser oder einer anderen Flüssigkeit vom Futterstrom entfernt positioniert ist, und das/die ferner Beförderungsmittel zum Befördern losgelöster Mikroorganismen an den Futterstrom umfasst.

4. Vorrichtung nach Anspruch 2 oder 3, die ferner Mittel zum Einstellen des Stroms von Wasser oder einer anderen Flüssigkeit an der Formulierung vorbei umfasst.

5. Vorrichtung nach den Ansprüchen 2-4, worin der Strom von Wasser oder einer anderen Flüssigkeit eine Nährbouillon umfasst.

6. Verfahren oder Vorrichtung nach einem der vorangehenden Ansprüche, worin die Mikroorganismen durch den Strom von Wasser oder einer anderen Flüssigkeit an der Formulierung vorbei aus der Formulierung losgelöst werden.

7. Verfahren oder Vorrichtung nach Anspruch 1 oder 2, worin die Mikroorganismen durch Inkontaktbringen der Formulierung mit dem Futterstrom losgelöst werden.

8. Verfahren oder Vorrichtung nach den Ansprüchen 1, 2 oder 7, worin der Futterstrom einen flüssigen Futterstrom darstellt.

9. Verfahren oder Vorrichtung nach einem der vorangehenden Ansprüche, worin die Mikroorganismen in einer gepulsten oder im Wesentlichen kontinuierlichen Weise von der Matrix losgelöst werden.

10. Verfahren oder Vorrichtung nach einem der vorangehenden Ansprüche, worin der Futterstrom Trinkwasser für das Tier umfasst.

11. Verfahren oder Vorrichtung nach einem der vorangehenden Ansprüche, worin der Futterstrom und/oder der Strom von Wasser oder einer anderen Flüssigkeit kontinuierlich oder halbkontinuierlich zugeführt wird/werden,

12. Verfahren oder Vorrichtung nach einem der vorangehenden Ansprüche, worin der Mikroorganismus einen probiotischen Organismus darstellt, der einen rekombinanten probiotischen Organismus einschließt.

13. Verfahren oder Vorrichtung nach einem der vorangehenden Ansprüche, worin die Formulierung ferner ein Präbiotikum umfasst.

14. Verfahren oder Vorrichtung nach Anspruch 13, worin das Präbiotikum aus der Gruppe ausgewählt ist, die aus einem unverdaulichen Oligosaccharid, Inulin, Cellulose, resistenter Stärke und Xylan aus Haferspelzen besteht.

15. Verfahren oder Vorrichtung nach Anspruch 13 oder 14, worin das Präbiotikum in einer Konzentration von ca. 2 % (G/V) vorliegt.

16. Verfahren oder Vorrichtung nach einem der vorangehenden Ansprüche, worin der Mikroorganismus einen *Lactobaeillus* oder einen *Enterococcus* darstellt.

17. Verfahren oder Vorrichtung nach einem der vorangehenden Ansprüche, worin der Mikroorganismus aus *L. acidophilus* LA-107 (ATCC-Nr. 53545), *L. acidophilus* LA-101 (ATCC-Nr. 4356) und *E. faecium* EF-101 (ATCC-Nr. 19434) ausgewählt ist.

18. Verfahren oder Vorrichtung nach einem der vorangehenden Ansprüche, worin die Formulierung mehr als einen Stamm oder eine Spezies von einem Mikroorganismus umfasst.

19. Verfahren oder Vorrichtung nach einem der vorangehenden Ansprüche, worin die Matrix ein wasserdurchlässiges Material umfasst.

20. Verfahren oder Vorrichtung nach einem der vorangehenden Ansprüche, worin die Matrix Alginat, bevorzugt Calciumalginat oder Bariumalginat umfasst.

21. Verfahren oder Vorrichtung nach einem der vorangehenden Ansprüche, worin die Formulierung im Wesentlichen wasserfrei ist.

22. Verfahren oder Vorrichtung nach einem der vorangehenden Anspruch, worin die Matrix der Gefriertrocknung, Sprühtrocknung oder Lyophilisierung unterzogen worden ist.

23. Verfahren oder Vorrichtung nach einem der vorangehenden Ansprüche, worin die Matrix ein Konservierungsmittel oder Kryokonservierungsmittel zur Erhaltung der Lebensfähigkeit der Mikroorganismen umfasst.

24. Verfahren oder Vorrichtung nach Anspruch 23, worin das Konservierungsmittel oder Kryokonservierungsmittel Trehalose oder Lactose oder eine Kombination aus Trehalose und Lactose umfasst.

25. Verfahren oder Vorrichtung nach Anspruch 23 oder 24, worin das Konservierungsmittel oder Kryokonservierungsmittel Lactose umfasst.

26. Verfahren oder Vorrichtung nach einem der vorangehenden Ansprüche, worin die Formulierung in einer Zuführungsvorrichtung bereitgestellt ist.

27. Verfahren oder Vorrichtung nach Anspruch 26, worin die Zuführungsvorrichtung eine Kartusche darstellt.

28. Verfahren oder Vorrichtung nach Anspruch 27, worin die Kartusche ein Maschensystem zum Zurückhalten der Formulierung in der Form von Perlen in der Kartusche umfasst, aber im Wesentlichen zulässt, dass die losgelösten Mikroorganismen mit dem Futterstrom fortgeschleppt werden.

## Revendications

1. Méthode d'administration d'un microorganisme à un courant d'alimentation pour une ingestion ultérieure par un animal, la méthode comprenant les étapes consistant à :
(a) fournir une formulation comprenant un microorganisme en suspension dans ou sur une matrice ;
(b) fournir un courant d'alimentation pour l'animal ;
(c) fournir un écoulement d'eau ou d'un autre liquide pour la formulation, où le courant d'alimentation et l'écoulement d'eau ou d'un autre liquide peut être le même courant, ou l'écoulement d'eau ou d'un autre liquide peut être positionné à une certaine distance du courant d'alimentation ;
(d) détacher les microorganismes de la matrice, et entraîner les microorganismes détachés dans l'écoulement d'eau ou d'un autre liquide ; et
(e) permettre à un animal d'ingérer le courant d'alimentation et l'écoulement d'eau ou d'un autre liquide.

2. Appareil d'administration d'un microorganisme à un courant d'alimentation pour une ingestion ultérieure par un animal, l'appareil comprenant :
(a) une formulation comprenant un microorganisme en suspension dans ou sur une matrice ;
(b) des moyens pour fournir un courant d'alimentation pour l'animal ;
(c) des moyens pour fournir un écoulement d'eau ou d'un autre liquide pour la formulation, où le courant d'alimentation et l'écoulement d'eau ou d'un autre liquide peut être le même courant, ou l'écoulement d'eau ou d'un autre liquide peut être positionné à une certaine distance du courant d'alimentation ;
(d) des moyens pour détacher les microorganismes de la matrice; et des moyens pour entraîner les microorganismes détachés dans l'écoulement d'eau ou d'un autre liquide ; et
(e) des moyens pour permettre à un animal d'ingérer le courant d'alimentation et l'écoulement d'eau ou d'un autre liquide.

3. Méthode ou appareil selon les revendications 1 ou 2, dans laquelle ou lequel l'écoulement d'eau ou d'un autre liquide est positionné à une certaine distance du courant d'alimentation, et comprenant en outre des moyens de transport destinés à transporter des microorganismes détachés vers le courant d'alimentation.

4. Appareil selon les revendications 2 ou 3, comprenant en outre des moyens pour ajuster l'écoulement d'eau ou d'un autre liquide au-delà de la formulation.

5. Appareil selon les revendications 2-4, dans lequel l'écoulement d'eau ou d'un autre liquide comprend un brouillon nutritif.

6. Méthode ou appareil selon l'une quelconque des revendications précédentes,
dans laquelle ou lequel les microorganismes sont détachés de la formulation par l'écoulement d'eau ou d'un autre liquide au-delà de la formulation.

7. Méthode ou appareil selon les revendications 1 ou 2, dans laquelle ou lequel les microorganismes sont détachés par la mise en contact de la formulation avec le courant d'alimentation.

8. Méthode ou appareil selon les revendications 1, 2 ou 7, dans laquelle ou lequel le courant d'alimentation est un courant d'alimentation liquide.

9. Méthode ou appareil selon l'une quelconque des revendications précédentes,
dans laquelle ou lequel les microorganismes sont détachés de la matrice de manière pulsée ou sensiblement continue.

10. Méthode ou appareil selon l'une quelconque des revendications précédentes,
dans laquelle ou lequel le courant d'alimentation comprend de l'eau de boisson pour l'animal.

11. Méthode ou appareil selon l'une quelconque des revendications précédentes,
dans laquelle ou lequel le courant d'alimentation et/ou l'écoulement d'eau ou d'un autre liquide est administré de manière continue ou semi-continue.

12. Méthode ou appareil selon l'une quelconque des revendications précédentes,
dans laquelle ou lequel le microorganisme est un organisme probiotique, y compris un organisme probiotique recombiné.

13. Méthode ou appareil selon l'une quelconque des revendications précédentes,
dans laquelle ou lequel la formulation comprend en outre un prébiotique.

14. Méthode ou appareil selon la revendication 13, dans laquelle ou lequel le prébiotique est choisi parmi le groupe constitué par : un oligosaccharide non digestible, l'inuline, la cellulose, l'amidon résistant, et le xylane d'épeautre.

15. Méthode ou appareil selon les revendications 13 ou 14, dans laquelle ou lequel le prébiotique est présent en une concentration d'environ 2% (p/v).

16. Méthode ou appareil selon l'une quelconque des revendications précédentes,
dans laquelle ou lequel le microorganisme est un *Lactobacillus* ou un *Enterococcus.*

17. Méthode ou appareil selon l'une quelconque des revendications précédentes, dans laquelle ou lequel le microorganism est choisi parmi *L. acidophilus* LA-107 (ATCC n°53545), *L. acidophilus* LA-101 (ATCC n°4356) *et E. faecium* EF-101 (ATCC n° 19434).

18. Méthode ou appareil selon l'une quelconque des revendications précédentes,
dans laquelle ou lequel la formulation comprend plus d'une souche ou espèce de microorganisme.

19. Méthode ou appareil selon l'une quelconque des revendications précédentes,
dans laquelle ou lequel la matrice comprend un matériau poreux à l'eau.

20. Méthode ou appareil selon l'une quelconque des revendications précédentes,
dans laquelle ou lequel la matrice comprend de l'alginate, préférablement de l'alginate de calcium ou de l'alginate de baryum.

21. Méthode ou appareil selon l'une quelconque des revendications précédentes,
dans laquelle ou lequel la formulation est sensiblement dépourvue d'eau.

22. Méthode ou appareil selon l'une quelconque des revendications précédentes,
dans laquelle ou lequel la matrice a été soumise à une lyophilisation ou un séchage par pulvérisation.

23. Méthode ou appareil selon l'une quelconque des revendications précédentes,
dans laquelle ou lequel la matrice comprend un conservateur ou un cryoprotecteur afin de maintenir la viabilité des microorganismes.

24. Méthode ou appareil selon la revendication 23, dans laquelle ou lequel le conservateur ou le cryoprotecteur comprend du tréhalose ou du lactose, ou une association de tréhalose et de lactose.

25. Méthode ou appareil selon les revendications 23 ou 24, dans laquelle ou lequel le conservateur ou le cryoprotecteur comprend du lactose.

26. Méthode ou appareil selon l'une quelconque des revendications précédentes,
dans laquelle ou lequel la formulation est fournie dans un dispositif d'administration,

27. Méthode ou appareil selon la revendication 26, dans laquelle ou lequel le dispositif d'administration est une cartouche.

28. Méthode ou appareil selon la revendication 27, dans laquelle ou lequel la cartouche comprend une grille afin de retenir la formulation sous la forme de billes au sein de la cartouche, mais permettant sensiblement aux microorganismes détachés d'être emportés avec le courant d'alimentation.
